# FASCICULE DE BREVET EUROPEEN

(11) **EP 0 983 287 B1**
(45) Date de publication et mention de la délivrance du brevet: **12.03.2003**
(21) Numéro de dépôt: 98925761.3
(22) Date de dépôt: 20.05.1998
(51) Int. Cl.: C07H 17/04, A61K 31/70

(54) **NOUVEAUX DERIVES GLYCOSYLES DES GINKGOLIDES, LEUR APPLICATION EN TANT QUE MEDICAMENTS ET COMPOSITIONS PHARMACEUTIQUES LES CONTENANT**
GLYKOSYLIERTE-GINKGOLID-DERIVATE, IHRE VERWENDUNG ALS ARZNEIMITTELN UND PHARMAZEUTISCHE ZUBEREITUNGEN DIE SIE ENTHALTEN
NOVEL GLYCOSYLATED GINKGOLIDE DERIVATIVES, THEIR APPLICATION AS MEDICINES AND PHARMACEUTICAL COMPOSITIONS

(30) Priorité: 20.05.1997 FR 9706111
(43) Date de publication de la demande: 08.03.2000
(73) Titulaire: SOCIETE DE CONSEILS DE RECHERCHES ET D'APPLICATIONS SCIENTIFIQUES (S.C.R.A.S.), 75016 Paris (FR)
(72) Inventeur: VASELLA, Andrea, CH-8057 Zürich (CH); WEBER, Martin, CH-8057 Zürich (CH)
(74) Mandataire: Bourgouin, André
(86) Numéro de dépôt international: FR9801016
(87) Numéro de publication internationale: WO98052959

(56) Documents cités:
- DE-A- 3 514 054
- FR-A- 2 622 448

## Description

La présente invention concerne de nouveaux dérivés glycosylés des ginkgolides, ainsi que leur application en tant que médicaments. De tels dérivés peuvent notamment être utilisés dans des compositions pharmaceutiques.

Les propriétés des ginkgolides (activité anti-PAF ("*Platelet Activating Factor*"), inhibition de la formation de radicaux libres, inhibition de la libération de glucocorticoïdes, etc.) sont connues (cf. notamment demandes de brevet DE-A 35 14 054, EP O 431 535 ; Amri, Ogwuegbu, Boujrad, Drieu, Papadopoulos, *Endocrinology,* **137**(12), 5707-5718). Cependant leur activité pharmaceutique est limitée par leur faible solubilité dans l'eau. La demanderesse a constaté que la glycosylation de ces ginkgolides conduit à des produits nouveaux hydrosolubles, donc plus facilement utilisables pour des compositions pharmaceutiques, tout en conservant l'activité biologique initiale des ginkgolides voire en l'améliorant.

L'invention concerne notamment des dérivés mono-, di-, ou, le cas échéant tri- ou tétraglycosylés, des ginkgolides A, B, C, J ou M (structures données dans le schéma ci-dessous ; ces composés peuvent être isolés à partir d'extraits de feuilles de *Ginkgo biloba -* voir *GINKGOLIDES, Chemistry, Biology, Pharmacology and Clinical Perspectives,* Edité par P. Braquet, J.R. Prous Science Publishers, notamment Volumes 1 (1988) et 2 (1989)). Elle concerne également des dérivés glycosylés de dérivés alkoxylés de ginkgolides, c'est à dire ceux comportant au moins un groupe alkoxy, linéaire ou ramifié, à la place d'un groupe hydroxy (ces composés sont décrits dans la demande de brevet français FR 88.14392).

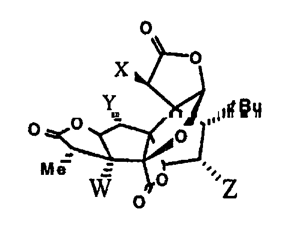

| *Structure des ginkgolides A, B C, J et M* | | | | |
|---|---|---|---|---|
| Ginkgolide | W | X | Y | Z |
| A | OH | OH | H | H |
| B | OH | OH | OH | H |
| C | OH | OH | OH | OH |
| J | OH | OH | H | OH |
| M | H | OH | OH | OH |

L'invention concerne un composé de formule générale (**I**) 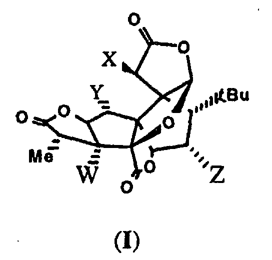 dans laquelle W, X, Y et Z représentent indépendamment les radicaux H, OH, alkoxy linéaire ou ramifié ou O-G_{S}, G_{S}-OH représentant un mono- ou un disaccharide, ou un de leurs dérivés ou analogues,
étant entendu que l'un au moins de W, X, Y ou Z représente un radical O-G_{S}.

L'invention concerne de préférence un composé de formule générale (**I**) 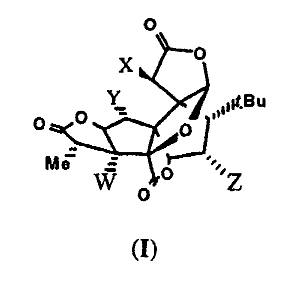 dans laquelle X représente un radical OH ou O-G_{S}, G_{S}-OH représentant un mono- ou un disaccharide, ou un de leurs dérivés ou analogues, et :
- ou bien W représente un radical OH ou O-G_{S}, Y représente H et Z représente H ;
- ou bien W représente un radical OH ou O-G_{S}, Y représente un radical OH ou O-G_{S} et Z représente H ;
- ou bien W représente un radical OH ou O-G_{S}, Y représente un radical OH ou O-G_{S} et Z représente un radical OH ou O-G_{S} ;
- ou bien W représente un radical OH ou O-G_{S}, Y représente H et Z représente un radical OH ou O-G_{S} ;
- ou bien W représente H, Y représente un radical OH ou O-G_{S} et Z représente un radical OH ou O-G_{S} ;
- ou bien W représente un radical OH ou O-G_{S}, Y représente un radical alkoxy linéaire ou ramifié et Z représente H ;
étant entendu que l'un au moins de W, X, Y ou Z représente un radical O-G_{S}.

L'invention concerne tout particulièrement un composé de formule générale (**I**) 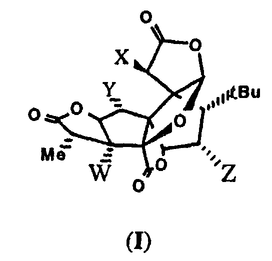 dans laquelle X représente un radical OH ou O-G_{S}, G_{S}-OH représentant un mono- ou un disaccharide ou un de leurs dérivés ou analogues, et :
- ou bien W représente un radical OH ou O-G_{S}, Y représente H et Z représente H ;
- ou bien W représente un radical OH ou O-G_{S}, Y représente un radical OH ou O-G_{S} et Z représente H ;
- ou bien W représente un radical OH ou O-G_{S}, Y représente un radical alkoxy linéaire ou ramifié et Z représente H ;
étant entendu que l'un au moins de W, X, Y ou Z représente un radical O-G_{S}.

Par radical alkoxy linéaire ou ramifié, on entend dans la présente description un radical alkoxy dont la chaine carbonée, linéaire ou ramifiée, compte de 1 à 6 atomes de carbone. Par dérivé ou analogue des mono- ou disaccharides, on entend des composés comme la N-acétylglucosamine, la N-acétylalosamine, la galactosamine, la mannoseamine, la N-tosylhydrazone, etc.

De préférence, O-G_{S} sera choisi de telle sorte que G_{S}-OH appartienne au groupe composé de l'abéquose, du rhamnose, de l'arabinose, du ribose, du xylose, du 2-déoxy-ribose, du glucose, du galactose, du mannose, du 2-déoxyglucose, du fructose, du fucose, de la N-acétylglucosamine, de la N-acétylalosamine, de la galactosamine, de la mannosamine, du saccharose, du lactose, du maltose, du cellobiose et du tréhalose. De façon encore plus préférentielle, O-G_{S} sera choisi de telle sorte que G_{S}-OH appartienne au groupe composé du glucose et du lactose.

L'invention concerne donc notamment des dérivés glycosylés des ginkgolides, plus particulièrement ceux des ginkgolides A et B, les groupes glycosyle adaptés pour l'invention étant décrits précédemment.

L'invention offre également un procédé pour obtenir les dérivés glycosylés des ginkgolides ou des ginkgolides alkoxylés (c'est à dire ceux résultant d'une réaction de glycosylation effectuée sur au moins un des groupes OH des ginkgolides ou de leurs dérivés alkoxylés). Ce procédé comprend principalement une étape de glycosylation, laquelle consiste en une réaction d'un composé de formule générale (**III**) représentée ci-dessous, 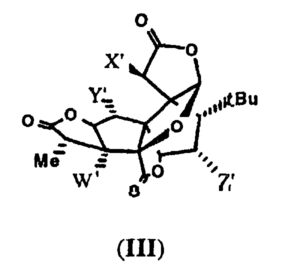 dans laquelle W', X', Y' et Z' représentent indépendamment un radical H, OH, alkoxy linéaire ou ramifié ou O-G_{X}, G_{X} étant un groupe protecteur d'un groupe hydroxy pouvant de préférence être éliminé en milieu neutre ou basique, étant entendu que l'un au moins de W', X', Y' et Z' représente OH,
avec une glycosyl diazirine de formule générale (**II**) représentée ci-dessous, la réaction s'effectuant de préférence dans du THF à des températures de préférence comprises entre 20 et 60 °C.

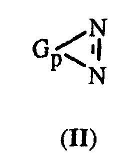

Le composé (**II**) est une diazirine dérivée d'un sucre Gₚ-OH dont tous les groupes hydroxy sauf celui porté par le carbone anomérique ont été protégés, par exemple par des radicaux benzyle ou silyle, tandis que le groupe hydroxy en position anomérique et l'hydrogène porté par le même atome de carbone ont été substitués par un groupe azi.

Ledit procédé peut également comprendre une ou plusieurs étapes de protection et/ou déprotection de groupes hydroxy. Pour ces étapes, l'homme du métier utilisera les méthodes classiques à sa disposition (Greene, T., Protective Groups in Organic Synthesis 10-86 (John Wiley & Sons 1981)) et il choisira de préférence des groupes protecteurs qui peuvent être éliminés en milieu basique ou neutre.

La réaction de glycosylation donnera généralement, dans le cas où un excès de glycosyldiazirine est employé, un mélange des dérivés mono- et diglycosylés pour le gingkolide A ou un mélange des dérivés mono-, di- et triglycosylés pour le ginkgolide B.

Dans tous les cas où on obtient un mélange de produits, on procédera à une séparation de ces produits selon les méthodes connues de l'homme du métier (notamment, filtration sur silice ou chromatographie liquide haute performance avec un éluant adapté, ou encore cristallisation ou recristallisation dans un solvant adéquat).

Les sucres ou dérivés de sucres utilisables pour la glycosylation des ginkgolides peuvent être notamment l'abéquose, le rhamnose, l'arabinose, le ribose, le xylose, le 2-déoxy-ribose, le glucose, le galactose, le mannose, le 2-déoxyglucose, le fructose, le fucose, le saccharose, le lactose, le maltose, le cellobiose, le tréhalose la N-acétylglucosamine, la N-acétylallosamine, la galactosamine, la mannosamine, ou des dérivés de ces composés, notamment les diazirines correspondantes, ou encore des N-arylsulfonylhydrazones comme la N-tosylhydrazone et leurs sels.

Lorsque la méthode employée est l'addition de glycosyl diazirines (méthode générale décrite dans Briner, K., Vasella, A., *Helv. Chim. Acta*, **72,** 1371 (1989)), on pourra notamment utiliser les diazirines suivantes :
- 1-azi-2,3,4,6-tétra-O-benzyl-1-déoxy-D-glucopyranose ou 1-azi-1-déoxy-2,3:5,6-di-O-isopropylidène-D-mannofurannose (synthèses décrites dans Briner, K., Vasella, A., *Helv. Chim. Acta*, **75**(2), 621-637 (1992)) ;
- 1-azi-2,3,4,6-tétra-O-benzyl-1-déoxy-D-galactopyranose (synthèse décrite dans Briner, K., Vasella, A., *Helv. Chim. Acta*, **73**(6), 1764-1779 (1990)) ;
- 1-azi-2,3,4,6-tétra-O-benzyl-1-déoxy-D-mannopyranose (synthèse décrite dans Vasella, A., Witzig, C., Waldraff, C., Uhlmann, P., Briner, K., et al., *Helv. Chim. Acta*, **76**(8), 2847-2875 (1993)) ;
- 2-acétamido-1,5-anhydro-1-azi-3-O-benzyl-4,6-O-benzylidène-1,2-didéoxy-D-allitol (synthèse décrite dans Vasella, A., Dhar, P., Witzig, C., *Helv. Chim. Acta*, **76**(4), 1767-1778 (1993) et Linden, A., Vasella, A., Witzig, C., *Helv. Chim. Acta*, **75**(5), 1572-1577 (1992)) ;
- 1,5-anhydro-1-azi-2-déoxy-3,4,6-tri-O-pivaloyl-D-glucitol (synthèse décrite dans Takahashi, Y., Vasella, A., *Helv. Chim. Acta,* **75**(5), 1563-1571 (1992)) ;
- 1,5-anhydro-1-azi-2,3,4,6-tétra-O-pivaloyl-D-glucitol ou 2-acétamido-1,5-anhydro-1-azi-3-O-benzyl-4,6-O-benzylidène-1,2-didéoxy-D-glucitol (synthèses décrites dans Vasella, A., Witzig, C., Waldraff, C., Uhlmann, P., Briner, K., et al., *Helv. Chim. Acta,* **76**(8), 2847-2875 (1993)) ;
- 1,5-anhydro-1-azi-2,3-di-O-benzyl-4,6-O-benzylidène-D-mannitol (synthèse décrite dans Uhlmann, P., Briner, K., et al., *Helv. Chim. Acta,* **76**(8), 2847-2875 (1993)) ;
- 1,5-anhydro-1-azi-2,3-di-O-benzyl-4,6-O-(4-méthoxybenzylidène)-D-glucitol (synthèse décrite dans Vasella, A., Witzig, C., Waldraff, C., Uhlmann, P., Briner, K., et al., *Helv. Chim. Acta,* **76**(8), 2847-2875 (1993)) ;
- 2-acétamido-1,5-anhydro-1-azi-3,4,6-tri-O-benzyl-2-déoxy-D-glucitol (synthèse décrite dans Vasella, A., Witzig, C., Waldraff, C., Uhlmann, P., Briner, K., et al., *Helv. Chim. Acta,* **76**(8), 2847-2875 (1993)) ;
- 1-azi-1-déoxy-2,3:4,6-di-O-isopropylidène-D-glucopyranose (synthèse décrite dans Uhlmann, P., Harth, E., Naughton, A.B., Vasella, A., *Helv. Chim. Acta*, **77**(8), 2335-2340 (1994)) ;
- (Z)-N'-2,3,5-tri-O-benzyl-D-(ribofuranosylidène)toluène-4-sulfonohydrazide ou (Z)-N'-2,3,5-tri-O-benzyl-D-(arabinofuranosylidène)toluène-4-sulfonohydrazide (synthèse décrite dans Mangholz, S.E., Vasella, A., *Helv. Chim. Acta*, **78**(4), 1020-1035 (1995)) ;
- 1-azi-2,3,6-tri-O-benzyl-4-O-[2,3,4,6-tétra-O-benzyl-D-galactopyranosyl]1-déoxy-D-glucopyranose (synthèse selon Briner, K., Vasella, A., *Helv. Chim. Acta*, **72,** 1371 (1989)).

L'homme du métier pourra bien sûr choisir d'autres composés du même type s'il le juge nécessaire.

Les solvants utilisables pour la réaction de glycosylation sont le 1,4-dioxane, le THF, le toluène, le chlorure de méthylène ou le 1,2-dichloroéthane. De préférence, on utilisera le THF.

Les conditions réactionnelles peuvent être des conditions thermiques ou photolytiques. Pour les conditions thermiques, on travaillera à des températures comprises de préférence entre 25 et 60° C. Pour les conditions photolytiques, on travaillera à basse température, typiquement entre -80° C et -60° C, avec généralement le THF pour solvant. Pour la photolyse, on peut par exemple utiliser une lampe à mercure haute pression *Philips HPK-125*. Typiquement, l'irradiation se fera sur une durée d'environ une heure, ou plus si nécessaire.

Pour les dérivés monoglucosylés du ginkgolide A, une voie de synthèse possible est la suivante :

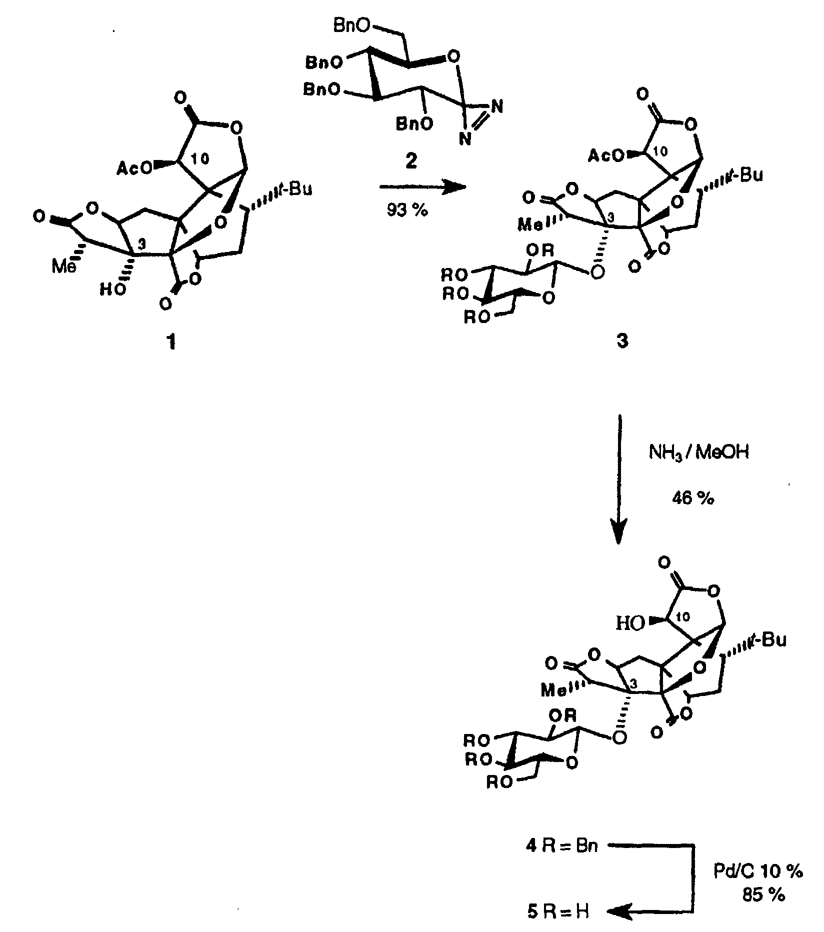

Le 10-O-acétyl-ginkgolide A (**1**) (préparé à partir du ginkgolide A que l'on fait réagir durant 25 heures à température ambiante avec de l'anhydride acétique en excès dans de la pyridine) est traité durant une heure et demie à 30° C par environ 1,3 équivalent de glucosyl diazirine **2,** la réaction étant effectuée dans le THF. Après évaporation et cristallisation, on obtient alors **3** qui est traité par NH₃ dans MeOH pour donner le produit désacétylé **4.** La débenzylation de **4** est ensuite effectuée de façon classique par hydrogénation avec Pd/C 10 % dans MeOH. On obtient alors le 3-O-(β-D-glucopyranosyl)ginkgolide A (**5**). On notera qu'on pourrait inverser les étapes de débenzylation et désacétylation.

De la même façon que l'on a réalisé la synthèse des dérivés monoglucosylés du ginkgolide A, on peut réaliser celle des dérivés monoglycosylés du ginkgolide A en utilisant d'autres glycosyl diazirines telles que celles décrites précédemment ou bien en réalisant dans des conditions classiques (décrites dans la littérature) la réaction de glycosylation.

A titre d'exemple, le 3-O-[(4-O-β-D-galactopyranosyl)-β-D-glucopyranosyl]ginkgolide A peut être obtenu par la voie suivante :

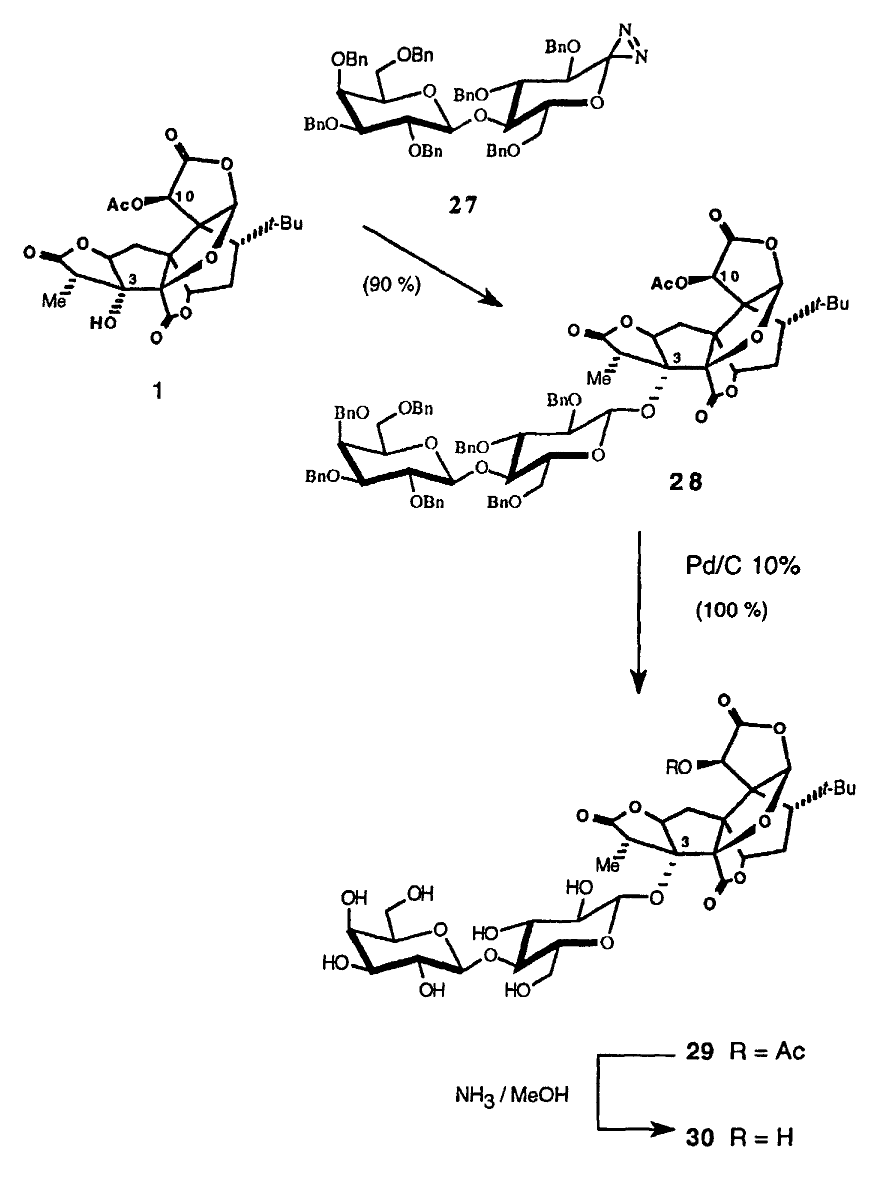

Le ginkgolide A (**1**) est traité par une solution de la diazirine **27** dans du THF durant une heure. On obtient alors **28** avec un rendement de 90 %. **28** est débenzylé par hydrogénation en présence de Pd/C 10 % pour donner **29.** Enfin, **29** peut être désacétylé par des méthodes classiques (par exemple NH₃/MeOH) pour donner le 3-O-[(4-O-β-D-galactopyranosyl)-β-D-glucopyranosyl]ginkgolide A (**30**). On peut bien sûr, comme précédemment, inverser les deux dernières étapes.

Si l'on souhaite obtenir le dérivé diglycosylé du ginkgolide A, il suffit de partir du ginkgolide A et non du 10-O-acétyl-ginkgolide A (**1**), et d'utiliser un excès de glycosyldiazirine ou de réactif glycosylant si l'on choisit une autre réaction de glycosylation.

Une voie de synthèse possible des dérivés du ginkgolide B selon l'invention est la suivante :

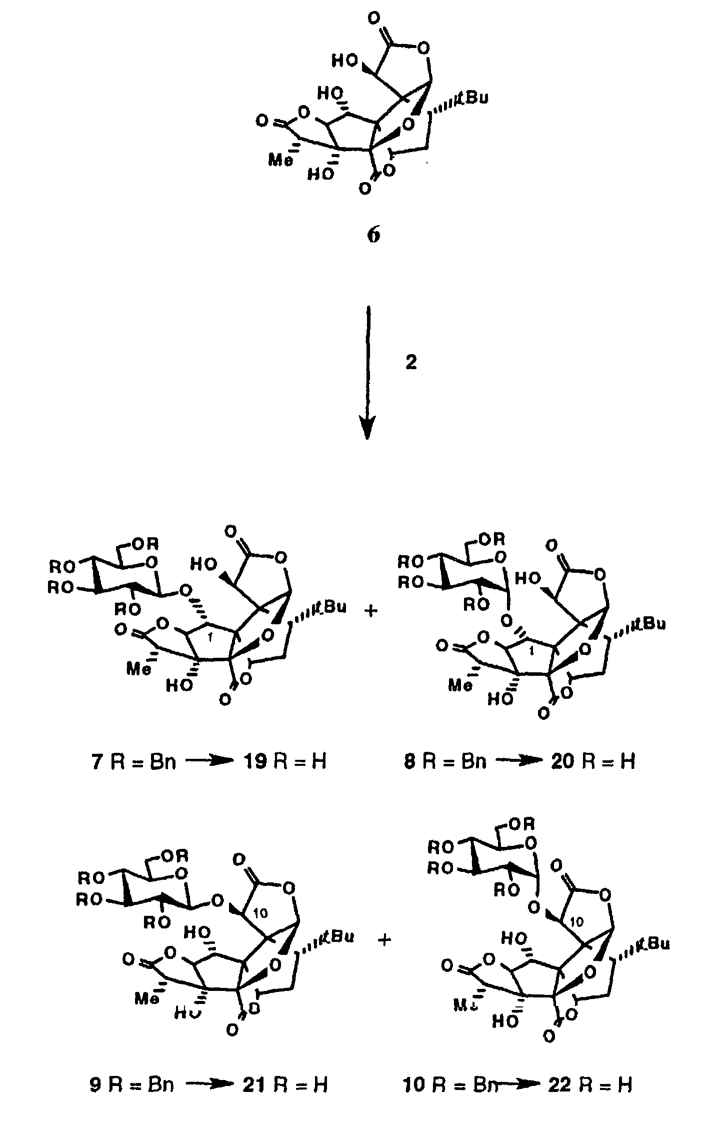

Pour obtenir les dérivés monoglucosylés du ginkgolide B (**6**), on choisira de préférence des conditions photolytiques pour la réaction de glycosylation, en utilisant de 1 à 1,35 équivalent de glucosyl diazirine **2** environ par équivalent de ginkgolide. La débenzylation des dérivés monoglycosylés **7, 8, 9** et **10** pour donner respectivement les composés **19, 20, 21** et **22** s'effectue comme décrit précédemment pour la débenzylation du composé **4.**

Les dérivés monoglycosilés du ginkgolide B s'obtiennent de la même façon en partant d'autres glycosyldiazirines que **2,** ou en partant d'un autre réactif glycosylant et en utilisant une réaction de glycosylation telle que celles décrites dans la littérature. Les étapes de déprotection suivant la réaction de glycosylation restent les mêmes.

Si l'on souhaite obtenir des dérivés diglucosylés du ginkgolide B, on pourra par exemple utiliser la voie de synthèse suivante :

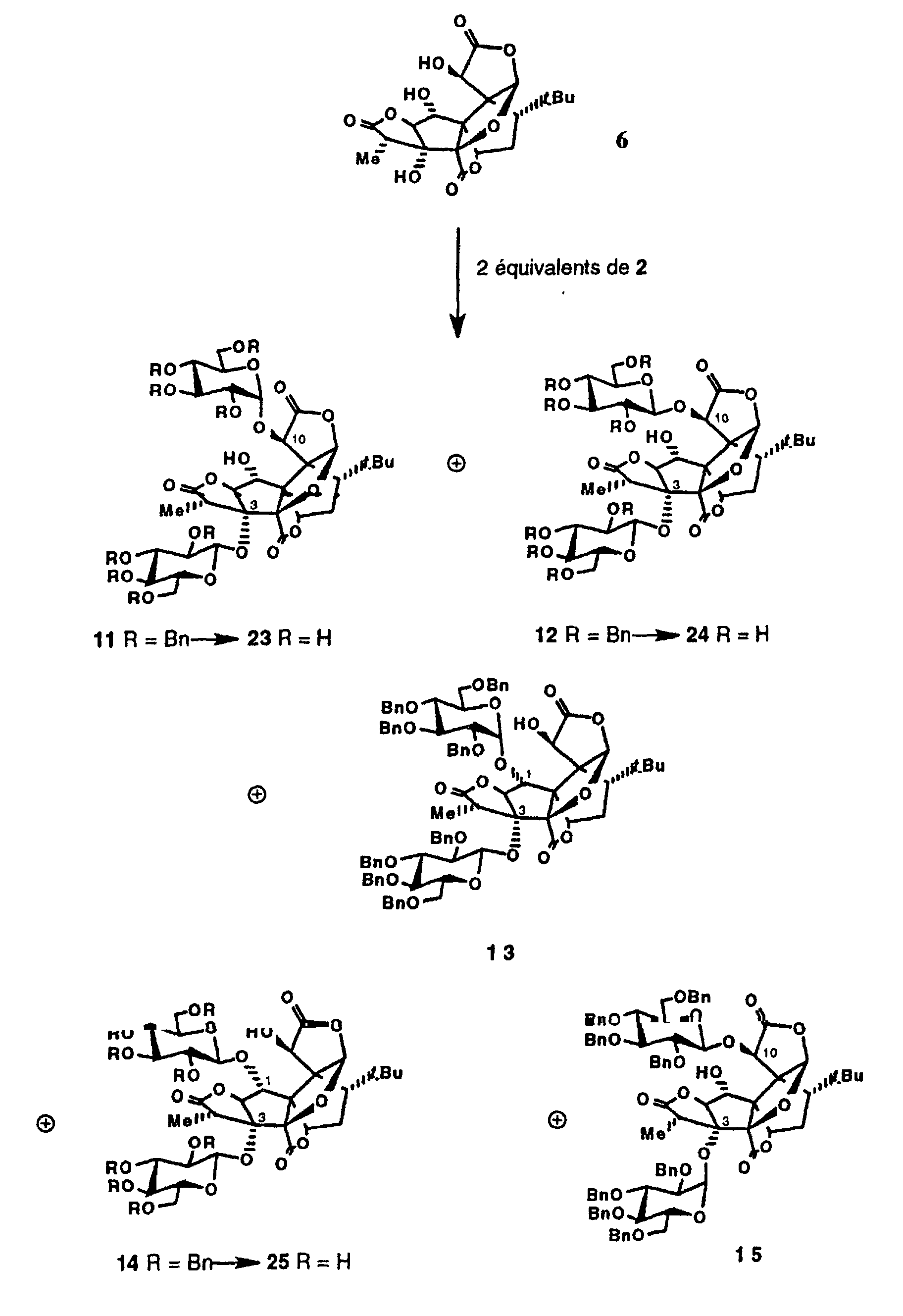

Pour obtenir les dérivés diglycosylés du ginkgolide B (**6**), on fait réagir **6** durant 1 heure à 30° C dans du THF avec 1 équivalent de glucosyl diazirine **2**, puis on ajoute encore 1 équivalent de glucosyl diazirine **2** et on laisse réagir dans les mêmes conditions durant **17** heures. On obtient alors un mélange des dérivés diglucosylés **11, 12, 13, 14** et **15.** Ces dérivés peuvent ensuite être débenzylés par hydrogénation avec Pd/C 10 % dans MeOH.

Les dérivés diglycosilés du ginkgolide B s'obtiennent de la même façon en partant d'autres glycosyldiazirines que **2**, ou en partant d'un autre réactif glycosylant et en utilisant une réaction de glycosylation telle que celles décrites dans la littérature. Les étapes de déprotection suivant la réaction de glycosylation restent les mêmes.

Pour obtenir les dérivés triglucosylés du ginkgolide B, on utilisera la voie de synthèse suivante :

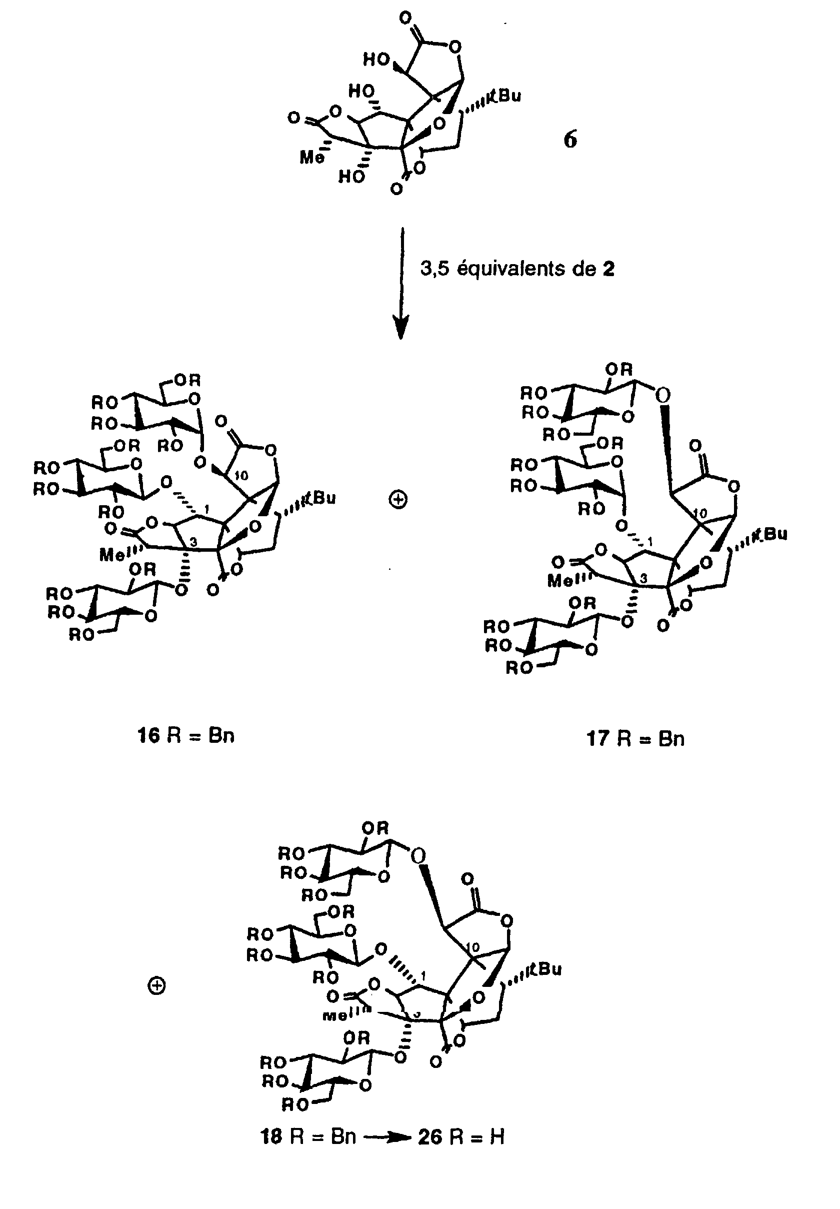

Les dérivés triglucosylés du ginkgolide B (**6**) s'obtiennent par réaction de **6** avec au moins 3,5 équivalents de glucosyl diazirine **2** dans du THF à 25° C, l'addition de **2** s'effectuant en 2 fois séparées par un intervalle de 24 heures par exemple. On laisse ensuite réagir pendant environ une journée et on obtient majoritairement le 1,3,10-O-(2,3,4,6-tétra-O-benzyl-β-D-glucopyranosyl)ginkgolide B (**18**). On obtient également le 1,3-O-(2,3,4,6-tétra-O-benzyl-β-D-glucopyranosyl)-10-O-(2,3,4,6-tétra-O-benzyl-α-D-glucopyranosyl)ginkgolide B (**16**) et le 1-O-(2,3,4,6-tétra-O-benzyl-α-D-glucopyranosyl)-3,10-O-(2,3,4,6-tétra-O-benzyl-β-D-glucopyranosyl)ginkgolide B (**17**). **18** peut ensuite être débenzylé par par hydrogénation avec Pd/C 10 % dans MeOH et on obtient alors le 1,3,10-O-(β-D-glucopyranosyl)ginkgolide B (**26**). Par la même méthode, on pourra débenzyler **16** et **17** pour obtenir les dérivés débenzylés correspondants.

Les dérivés triglycosylés du ginkgolide B s'obtiennent de la même façon en partant d'autres glycosyl diazirines que **2**, ou en partant d'un autre réactif glycosylant et en utilisant une réaction de glycosylation telle que celles décrites dans la littérature. Les étapes de déprotection suivant la réaction de glycosylation restent les mêmes.

Le ginkgolide C pourra être glycosylé de façon analogue à celle utilisée pour les ginkgolides A ou B. L'homme du métier choisira la stoechiométrie en glycosyl diazirine ou en réactif glycosylant en fonction du degré de glycosylation souhaité (dérivé mono-, di-, tri- ou tétraglycosylé).

De la même façon, les procédés décrits précédemment pourront être appliqués aux ginkgolides J et M pour donner des dérivés glycosylés de ces derniers, ou aux dérivés alkoxylés des gikgolides pour donner les composés glycosylés correspondants.

Les composés selon l'invention présentent d'intéressantes propriétés pharmacologiques et peuvent être par exemple utilisés pour traiter les troubles vasculaires, et en particulier les troubles cardiovasculaires. L'invention a donc également pour objet tous les dérivés glycosylés des ginkgolides A, B, C, J et M, ou les dérivés glycosylés des ginkgolides alkoxylés cités précédemment à titre de médicament, ainsi que des compositions pharmaceutiques comprenant, à titre de principe actif, au moins un de ces composés.

L'invention concerne ainsi des compositions pharmaceutiques contenant un composé de l'invention, en association avec un support pharmaceutiquement acceptable suivant le mode d'administration choisi (par exemple orale, intraveineuse, intrapéritonéale, intramusculaire, trans-dermique ou sous-cutanée). Ces compositions pharmaceutiques peuvent être sous forme solide, liquide, de liposomes ou de micelles lipidiques.

Les compositions pharmaceutiques selon l'invention peuvent être sous forme solide comme, par exemple, les poudres, pilules, granules, comprimés, liposomes, gélules ou suppositoires. La pilule, le comprimé ou la gélule peuvent être revêtus d'une substance capable de protéger la composition de l'action de l'acide gastrique ou des enzymes dans l'estomac du sujet pendant une période de temps suffisante pour permettre à cette composition de passer non digérée dans l'intestin grêle de ce dernier. Le composé peut aussi être administré selon le processus de la libération prolongée (par exemple une composition à libération prolongée ou une pompe d'infusion). Les supports solides appropriés peuvent être, par exemple, le phosphate de calcium, le stéarate de magnésium, le carbonate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire. Les compositions pharmaceutiques contenant un composé de l'invention peuvent donc également se présenter sous forme liquide comme, par exemple, des solutions, des émulsions, des suspensions ou une formulation à libération prolongée. Les supports liquides appropriés peuvent être, par exemple, l'eau, les solvants organiques tels que le glycérol ou les glycols tel que le polyéthylène glycol, de même que leurs mélanges, dans des proportions variées, dans l'eau.

L'invention concerne en outre l'utilisation d'un de ces dérivés pour fabriquer un médicament destiné à traiter les maladies vasculaires, en particulier les maladies cardiovasculaires.

La dose d'un composé selon la présente invention, à prévoir pour le traitement des maladies ou troubles mentionnés ci-dessus, varie suivant le mode d'administration, l'âge et le poids corporel du sujet à traiter ainsi que l'état de ce dernier, et il en sera décidé en définitive par le médecin ou le vétérinaire traitant. Une telle quantité déterminée par le médecin ou le vétérinaire traitant est appelée ici "quantité thérapeutiquement efficace".

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

Les exemples suivants sont présentés pour illustrer les procédures ci-dessus et ne doivent en aucun cas être considérés comme une limite à la portée de l'invention.

### EXEMPLES :

Pour tous les exemples qui suivent, les produits déjà décrits gardent les numéros tels qu'ils ont été indiqués plus haut. Les déplacements des signaux RMN sont donnés en ppm.

### Exemple 1 : 3-O-(β-D-glucopyranosyl)ginkgolide A (5) :

### 1.1. 10-O-acétyl-ginkgolide A (1) :

Du ginkgolide A (85 mg, 0,208 mmol) dans de la pyridine (0,75 ml) a été traité avec Ac₂O (82 µl, 0,87 mmol) et a réagi pendant 20 heures à 25° C sous agitation. Après addition d'éthanol, évaporation, et chromatographie-éclair (éluant hexane/acétone 6:4), on a obtenu **1** (88 mg, 94 %).
IR (CHCl₃) : 3483, 2960, 1780, 1602, 1372, 1325, 1152, 1125, 1104, 1084, 1062, 994, 940, 899.
RMN ¹H (200 MHz, acétone (D₆)) : 6,29 (*s*, H-C(10)); 6,19 (*s*, H-C(12)); 5,31 (*s*, HO-C(3)); 5,04-5,01 (*m*, H-C(6)); 4,92 (*dd*, *J* = 7,5 Hz, 9,1 Hz, H-C(2)); 3,15 (*q*, *J* = 7,1 Hz, H-C(14)); 3,03 (*dd*, *J* = 7,5 Hz, 14,9 Hz, H-C(1)); 2,25 (*s*, AcO); 2,24-1,86 (*m*, H-C(1), H-C(7), H'-C(7), H-C(8)); 1,29 (*d*, *J* = 7,1 Hz, Me-C(14)); 1,12 (*s*, *t*Bu).

### 1.2. 10-O-acétyl-3-O-(2,3,4,6-tétra-O-benzyl-β-D-glucopyranosyl)ginkgolide A (3) :

**1** tel qu'obtenu par l'étape 1.1. (59 mg, 0,131 mmol), dans du THF (6,0 ml), a été traité par une solution de 1-azi-2,3,4,6-tétra-O-benzyl-1-déoxy-D-glucopyranose (**2**) (95 mg, 0,172 mmol) et agité pendant une heure et demie à 30° C. Après évaporation et cristallisation (mélange hexane/CH₂Cl₂/AcOEt), on a obtenu **3** (108,5 mg, 85 %). Par HPLC (hexane/AcOEt 4:1), on récupère encore à partir de la solution mère 9,8 mg de **3** (8 %).
P_{f} 222°C
IR (CHCl₃) : 3066, 3008, 2967, 2874, 1801, 1605, 1497, 1454, 1406, 1373, 1323, 1276, 1116, 1074, 997, 957, 900.
RMN ¹H (300 MHz, acétone (D₆)) : 7,44-7,22 (*m*, 20 H); 6,34 (*s*, H-C(10)) ; 6,21 (*s*, H-C(12)); 5,60 (*dd*, *J* = 8,4 Hz, 9,0 Hz, H-C(2)); 5,01 (*d*, *J* = 3,1 Hz, H-C(6)); 4,92 (*d*, *J* = 10,6 Hz), 4,82 (*s*), 4,77 (*d*, *J* = 10,9 Hz, 4 PhC*H*); 4,72 (*d*, *J* = 7,5 Hz, H-C(1')); 4,66 (*d*, *J* = 11,2 Hz), 4,65 (*d*, *J* = 11,5 Hz), 4,61 (*d*, *J* = 10,6 Hz), 4,51 (*d*, *J* = 11,8 Hz, 4 PhC*H*); 3,81-3,75 (*m*, H-C(4'), H-C(6'), H'-C(6')); 3,60 (*t*, *J* = 9,0 Hz, H-C(3')); 3,41 (*dd*, *J* = 7,5 Hz, 9,0 Hz, H-C(2')); 3,34 (*dt*, *J* = 2,2 Hz, 9,6 Hz, H-C(5')); 3,20 (*q*, *J* = 6,8 Hz, H-C(14)); 3,17 (*dd*, *J*= 7,5 Hz, 14,9 Hz, H-C(1)); 2,25 (*s*, AcO); 2,21-2,16 (*m*, 2 H); 2,01-1,89 (*m*, 2H); 1,43 (*d*, *J* = 6,8 Hz, Me-C(14)); 1,11 (*s*, *t*Bu).
RMN ¹³C (100 MHz, acétone (D₆)): 176,4 (*s*); 170,4 (*s*); 169,6 (*s*); 169,1 (*s*); 140,0 (*s*); 139,9 (*s*); 139,7 (2*s*); 129,1-128,1 (plusieurs *d*); 111,8 (*d*); 101,9 (*s*);
99,3 (*d*); 94,4 (*s*); 85,7 (*d*); 85,2 (*d*); 82,6 (*d*); 82,2 (*d*); 78,0 (*d*); 75,9 (*t*); 75,7 (*d*); 75,5 (*t*); 75,2 (*t*); 73,9 (*t*); 70,3 (*d*); 69,6 (*s*); 68,3 (*t*); 66,8 (*s*); 49,8 (*d*); 41,9 (*d*); 38,1 (*t*); 37,5 (*t*); 32,8 (*s*); 20,5 (*q*); 8,4 (*q*).
Analyse élémentaire : C 68,87 %, H 6,21 % (valeurs théoriques : C 69,12 % et H 6,21 %).

### 1.3. 3-O-(2,3,4,6-tétra-O-benzyl-β-D-glucopyranosyl)ginkgolide A (4) :

**3** tel qu'obtenu par l'étape 1.2. (110 mg, 0,11 mmol), placé dans MeOH (5 ml) et du CH₂Cl₂ (2 ml), a été traité par une solution saturée en NH₃ dans MeOH (1,2 ml) et agité à 25° C pendant une heure et demie. Après évaporation et chromatographie éclair (éluant hexane/acétone 3:2) et HPLC, on a obtenu 48 mg de **4** (46 %).
IR (CHCl₃) : 3290, 3008, 2967, 2874, 1794, 1497, 1454, 1362, 1324, 1144, 1073, 1028, 995, 953, 903.
RMN ¹H (300 MHz, acétone (D₆)) : 7,43-7,25 (*m*, 20 H); 6,13 (*s*, H-C(12)); 5,95 (*d*, *J* = 4,4 Hz, HO-C(10)); 5,54 (*t*, *J* = 8,4 Hz, H-C(2)); 5,22 (*d*, *J* = 4,1 Hz, H-C(10)); 4,95-4,91 (*m*, H-C(6), Ph-CH), 4,82 (*s*), 4,77 (*d*, *J* = 11,2 Hz, 3 PhC*H*); 4,72 (*d*, *J* = 7,5 Hz, H-C(1')); 4,65 (*d*,*J* = 13,1 Hz), 4,65 (*d*, *J* = 11,5 Hz), 4,61 (*d*, *J* = 10,9 Hz), 4,52 (*d*, *J* = 11,8 Hz, 4 PhC*H*); 3,81-3,74 (*m*, H-C(4'), H-C(6'), H'-C(6')); 3,61 (*t*, *J* = 9,0 Hz, H-C(3')); 3,43-3,33 (*m*, H-C(2'), H-C(5')); 3,21 (*q*, *J* = 6,8 Hz, H-C(14)); 3,03 (*dd*, *J* = 7,5 Hz, 14,9 Hz, H-C(1)); 2,25 (*s*, AcO); 2,30-1,91 (*m*, 4 H); 1,43 (*d*, *J* = 6,8 Hz, Me-C(14)); 1,15 (*s*, *t*Bu).

### 1.4. 3-O-(β-D-glucopyranosyl)ginkgolide A (5) :

**4** tel qu'obtenu par l'étape 1.3. (47 mg, 0,05 mmol), mélangé à Pd/C 10 % (90 mg), a été hydrogéné dans MeOH (10 ml) durant 72 heures sous une pression de 3 atm. Après filtration, évaporation, et chromatographie éclair (éluant AcOEt/MeOH/H₂O 8,5:1,5:1), on a obtenu **5** (25 mg, 84 %).
RMN ¹H (300 MHz, CD₃OD) : 6,07 (*s*, H-C(12)); 5,50 (*dd*, *J* = 8,1 Hz, 9,0 Hz, H-C(2)); 5,04 (*s*, H-C(10)); 4,90 (*d*, *J* = 3,4 Hz, H-C(6)); 4,50 (*d, J* = 7,5 Hz, H-C(1')); 3,75 (*dd*, *J* = 2,2 Hz, 12,1 Hz, H-C(6')); 3,60 (*dd*, *J* = 4,8 Hz, 12,1 Hz, H'-C(6')); 3,35-3,15 (*m*, 5H); 2,93 (*dd*, *J* = 7,5 Hz, 14,9 Hz, H-C(1)); 2,29-2,11 (*m*, 2H); 2,00-1,85 (*m*, 2 H); 1,33 (*d*, *J* = 6,8 Hz, Me-C(14)); 1,10 (*s*, *t*Bu).

### Exemple 2 : Traitement du ginkgolide B (6) avec 1, 2 ou 3,5 équivalents de 1-azi-2,3,4,6-tétra-O-benzyl-1-déoxy-D-glucopyranose (2) :

### 2.1. Traitement avec 1 équivalent d'azirine :

Du ginkgolide B (**6**) (134,0 mg, 0,31 mmol) dans du THF (4,0 ml) a été refroidi à -75° C, traité avec une solution de 1-azi-2,3,4,6-tétra-O-benzyl-1-déoxy-D-glucopyranose (**2**) (177 mg, 0,32 mmol) dans du THF (1,5 ml), et irradié (lampe à mercure haute pression *Philips HPK-125*) pendant 1 heure à -70° C. Le mélange a ensuite été réchauffé à 23° C et évaporé. Après filtration sur silice (éluant AcOEt) et HPLC (éluant hexane/AcOEt 2:1), on a obtenu **7** (140,3 mg, 47 %), **8** (23,0 mg, 8 %), **9** (82,4 mg, 27 %), et **10** (29,8 mg, 10 %).

### 2.2. Traitement avec 2 équivalents d'azirine :

Du ginkgolide B (**6**) (97,2 mg, 0,229 mmol), dans du THF (2,0 ml), a été traité par une solution de 1-azi-2,3,4,6-tétra-O-benzyl-1-déoxy-D-glucopyranose (**2**) (135 mg, 0,245 mmol) dans du THF (1,0 ml) à 30° C, puis, une heure après, par une deuxième solution de **2** (119,0 mg, 0,216 mmol) dans du THF (0,9ml). Le milieu réactionnel est ensuite agité pendant 17 heures à 30° C. Après évaporation, filtration sur silice (éluant AcOEt) et HPLC (éluant hexane/AcOEt 2:1), on a obtenu **7** (26,3 mg, 12 %), **9** (35,8 mg, 17 %), **10** (34,9 mg, 16 %), **11** (20,7 mg, 6 %), **12** (32,6 mg, 10 %), **13** (8,9 mg, 3 %), **14** (15,6 mg, 5 %), **15** (5,6 mg, 2 %), un mélange de **16** et **17** (8,1 mg, 2 %) et **18** (6,7 mg, 2 %).

### 2.3. Traitement avec 3,5 équivalents d'azirine :

Du ginkgolide B (**6**) (27,0 mg, 0,064 mmol) a été traité par une solution de 1-azi-2,3,4,6-tétra-O-benzyl-1-déoxy-D-glucopyranose (**2**) (60,0 mg, 0,109 mmol) dans du THF (1,3 ml) à 25° C, puis, 24 heures après, par une deuxième solution de **2** (60,0 mg, 0,109 mmol) dans du THF (1,3 ml). Le milieu réactionnel est ensuite agité pendant 20 heures à 25° C. Après évaporation, filtration sur silice (éluant AcOEt) et HPLC (éluant hexane/AcOEt 4:1), on a obtenu **11** (21,9 mg, 24 %), **16** (18,4 mg, 15 %), **17** (16,0 mg, 13 %) et **18** (51,0 mg, 42 %).

### 2.4. Données IR et RMN des produits 7 à 18 :

### 1-O-(2,3,4,6-tétra-O-benzyl-β-D-glucopyranosyl)ginkgolide B (7) :

IR (CHCl₃) : 3564, 3451, 3328, 3090, 3066, 3007, 2966, 2913, 2873, 1952, 1794, 1604, 1497, 1454, 1405, 1360, 1324, 1310, 1172, 1143, 1070, 1028, 962, 906.
RMN ¹H (500 MHz, acétone (D₆)) : 7,42-7,24 (*m*, 20 H); 6,02 (*s*, H-C(12)); 5,60 (*d*, *J* = 3,7 Hz, H-C(6)); 5,50 (*s*, échangé par D₂O, HO-C(3)); 4,98-4,96 (*m*, 2 H); 4,87 (*d*, *J* = 11,2 Hz, PhC*H*); 4,84-4,79 (*m*, 3 H); 4,76 (*d*, *J* = 11,3 Hz), 4,67 (*d*, *J* = 11,1 Hz), 4,66 (*d*, *J* = 12,3 Hz), 4,60 (*d*, *J* = 12,3 Hz, 4 PhC*H*); 3,83 (*dd*, *J* = 2,0 Hz, 11,0 Hz, H-C(6')); 3,78 (*dd*, *J* = 3,9 Hz, 11,0 Hz, H'-C(6')); 3,71 (*t*, *J* = 8,9 Hz, H-C(4')); 3,66 (*t*, *J* = 8,5 Hz, H-C(3')); 3,51 (*ddd*, *J* = 1,9 Hz, 3,9 Hz, 9,5 Hz, H-C(5')); 3,46 (*t*, *J* = 8,1 Hz, H-C(2')); 3,06 (*q*, *J* = 7,5 Hz, H-C(14)); 2,10 (*ddd*, *J* = 3,9 Hz, 13,7 Hz, 13,9 Hz, H-C(7)); 2,03 (*dd*, *J* = 4,7 Hz, 13,5 Hz, H'-C(7)); 1,93 (*dd*, *J* = 4,7 Hz, 14,0 Hz, H-C(8)); 1,29 (*d*, *J* = 7,5 Hz, Me-C(14)); 1,11 (*s*, *t*-Bu).
RMN ¹³C (125 MHz, acétone (D₆)) : 176,5 (*s*); 173,9 (*s*); 171,7 (*s*); 140,1 (*s*); 139,9 (*s*); 139,8 (*s*); 139,8 (*s*); 129,3-128,4 (plusieurs *d*) 110,1 (*d*); 104,0 (*d*); 103,3 (*s*); 95,5 (*d*); 86,2 (*s*); 85,8 (*d*); 83,7 (*d*); 83,4 (*d*); 80,2 (*d*); 79,8 (*d*); 76,1 (*t*); 76,0 (*d*); 75,4 (2*t*); 74,9 (*s*); 74,2 (*t*); 70,4 (*d*); 70,2 (*s*); 69,7 (*t*); 50,6 (*d*); 42,1 (*d*); 37,8 (*t*); 33,3 (*s*); 10,4 (*q*).

### 1-O-(2,3,4,6-tétra-O-benzyl-α-D-glucopyranosyl)ginkgolide B (8) :

IR (CHCl₃) : 3566, 3342, 3089, 3069, 3007, 2966, 2873, 1795, 1603, 1497, 1454, 1406, 1357, 1326, 1284, 1168, 1100, 1068, 1029, 986, 962, 902.
RMN ¹H (400 MHz, acétone (D₆)) : 7,34-7,18 (20 H); 6,08 (*s*, H-C(12)); 5,62 (*d*, *J* = 3,5 Hz, H-C(6)); 5,36 (*s* , échangé par D₂O, HO-C(3)); 4,93 (*d*, *J* = 11,2 Hz, PhC*H*), 4,87-4,81 (*m*, 5 H); 4,79 (*d*, *J* = 11,6 Hz), 4,60 (*d*, *J* = 12,2 Hz), 4,57 (*d*, *J* = 12,1 Hz, 3 PhC*H*); 4,21 (*dt*, *J* = 3,0 Hz, 10,1 Hz, H-C(5')); 3,90 (*t*, *J* = 9,4 Hz, H-C(3')); 3,70 (*d*, *J* = 3,1 Hz, 2 H-C(6')); 3,61 (*dd*, *J* = 3,7 Hz, 9,6 Hz, H-C(2')); 3,57 (*dd*, *J* = 9,1 Hz, 10,1 Hz, H-C(4')); 3,12 (*q*, *J* = 7,2 Hz, H-C(14)); 2,20 (*dd*, *J* = 5,2 Hz, 13,4 Hz, H-C(7)); 2,19-2,13 (*m*, H'-C(7)); 1,92 (*dd*, *J* = 5,2 Hz, 13,5 Hz, H-C(8)); 1,28 (*d*, *J* = 7,2 Hz, Me-C(14)); 1,14 (*s*, *t*-Bu).
RMN ¹³C (100 MHz, acétone (D₆)) : 176,2 (*s*); 173,9 (*s*); 171,1 (*s*); 140,1 (*s*); 139,7 (*s*); 139,5 (2*s*); 129,2-128,2 (plusieurs *d*); 110,5 (*d*); 101,0 (*s*); 96,3 (*d*); 93,7 (*d*); 85,4 (*s*); 82,3 (*d*); 81,1 (*d*); 80,1 (*d*); 79,8 (*d*); 78,9 (*d*); 75,7 (2*t*); 73,9 (*t*); 73,9 (*t*); 73,6 (*s*); 72,5 (*d*); 70,1 (*d*); 69,8 (*s*, *t*); 50,0 (*d*); 42,5 (*d*); 37,6 (*t*); 33,1 (*s*); 8,8 (*q*).

### 10-O-(2,3,4,6-tétra-O-benzyl-β-D-glucopyranosyl)ginkgolide B (9) :

IR (CHCl₃) : 3565, 3490, 3090, 3067, 3008, 2966, 2944, 2915, 2872, 1951, 1785, 1605, 1497, 1454, 1406, 1361, 1327, 1314, 1280, 1163, 1098, 1028, 988, 967, 926.

RMN ¹H (500 MHz, acétone (D₆)) : 7,51-7,08 (*m*, 20 H); 6,12 (*s*, H-C(12)); 5,36-5,35 (*m*, H-C(6)); 5,28 (*s*, échangé par D₂O, HO-C(3)); 5,04 (*d*, *J* = 11,8 Hz), 4,92 (*d*, *J* = 11,2 Hz), 4,81 (*d*, *J* = 10,3 Hz), 4,80 (*d*, *J* = 11,7 Hz), 4,79 (*d*, *J* = 11,0 Hz), 4,70 (*d*, *J* = 11,0 Hz), 4,69 (*d*, *J* = 12,0 Hz), 4,57 (*d*, *J* = 12,0 Hz, 8 PhC*H*); 3,87 (*t*, *J* = 8,6 Hz, H-C(3')); 3,83 (*dd*, *J* = 3,9 Hz, 11,2 Hz, H-C(6')); 3,80 (*t*, *J* = 8,7 Hz, H-C(4')); 3,71 (*dd*, *J* = 2,0 Hz, 11,5 Hz, H'-C(6')); 3,70-3,67 (*m*, H-C(5')); 3,64 (*dd*, *J* = 7,5 Hz, 8,4 Hz, H-C(2')); 3,09 (*q*, *J* = 7,0 Hz, H-C(14)); 2,05-1,96 (*m*, H-C(7), H-C(8)); 1,86-1,82 (*m*, H'-C(7)); 1,28 (*d*, *J* = 7,0 Hz, Me-C(14)); 1,06 (*s*, *t*-Bu).
RMN ¹³C (125 MHz, acétone (D₆)): 176,8 (*s*); 171,0 (*s*); 170,3 (*s*); 139,6 (*s*); 139,4 (*s*); 139,4 (*s*); 139,4 (*s*); 129,3-127,9 (plusieurs *d*); 110,3 (*d*); 100,5 (*d*); 99,7 (*s*); 93,5 (*d*); 85,9 (*d*); 83,9 (*s*); 81,6 (*d*); 79,5 (*d*); 78,7 (*d*); 75,9 (*d*); 75,6 (*t*); 75,3 (*t*); 75,2 (*d*); 74,9 (*d*); 74,4 (*t*); 74,2 (*t*); 73,3 (*s*); 69,4 (*t*); 69,0 (*s*); 50,1 (*d*); 42,9 (*d*); 37,6 (*t*); 32,8 (*s*); 8,2 (*q*).

### 10-O-(2,3,4,6-tétra-O-benzyl-α-D-glucopyranosyl)ginkgolide B (10) :

IR (CHCl₃) : 3566, 3388, 3090, 3067, 3008, 2964, 2916, 2874, 1952, 1790, 1604, 1497, 1454, 1406, 1363, 1326, 1312, 1165, 1103, 1069, 1028, 1005, 987, 966, 920.
RMN ¹H (400 MHz, acétone (D₆)) : 7,39-7,06 (20 H); 6,25 (*s*, H-C(12)); 5,42 (*d*, *J* = 3,9 Hz, H-C(6)); 5,31 (*s*, échangé par D₂O, HO-C(3)); 5,07 (*d*, *J* = 10,8 Hz), 4,83 (*d, J* = 10,7 Hz), 4,81 (*d*, *J* = 11,0 Hz), 4,77 (*d*, *J* = 11,2 Hz), 4,75 (*d*, *J* = 11,3 Hz), 4,62 (*d*, *J* = 10,7 Hz), 4,60 (*d*, *J* = 11,9 Hz), 4,52 (*d*, *J* = 11,9 Hz, 8 PhC*H*); 3,88-3,70 (*m*, 5 H); 3,55 (*dd*, *J* = 1,4 Hz, 10,7 Hz, H'-C(6')); 3,07 (*q*, *J*= 7,0 Hz, H-C(14)); 2,24 (*dd*, *J* = 4,3 Hz, 13,4 Hz, H-C(7)); 2,04-1,96 (*m*, H'-C(7)); 1,85 (*dd, J =* 4,1 Hz, 14,5 Hz, H-C(8)); 1,28 (*d, J* = 7,0 Hz, Me-C(14)); 1,18 (*s*, *t*-Bu).
RMN ¹³C (100 MHz, acétone (D₆)) : 176,6 (*s*); 172,9 (*s*); 171,0 (*s*); 139,6 (*s*); 139,5 (*s*); 139,4 (*s*); 137,6 (*s*); 129,4-128,3 (plusieurs *d*); 110,7 (*d*); 99,6 (*s*); 96,6 (*d*); 93,3 (*d*); 83,4 (*s*); 81,8 (*d*); 80,8 (*d*) 79,2 (*d*); 77,9 (*d*); 76,1 (*t*); 75,8 (*t*); 75,7 (*t*); 74,7 (*d*); 74,1 (*t*); 73,7 (*d*); 73,7 (*d*); 72,9 (*s*); 69,1 (*t*); 69,2 (*s*); 49,6 (*d*); 42,9 (*d*); 38,4 (*t*); 33,1 (*s*); 8,2 (*q*).

### 3-O-(2,3,4,6-tétra-O-benzyl-β-D-glucopyranosyl)-10-O-(2,3,4,6-tétra-O-benzyl-α-D-glucopyranosyl)ginkgolide B (11) :

IR (CHCl₃) : 3395, 3090, 3067, 3008, 2923, 2874, 1951, 1793, 1606, 1497, 1454, 1406, 1362, 1325, 1275, 1163, 1070, 1028, 987, 920, 902.
RMN ¹H (300 MHz, acétone (D₆)) : 7,46-7,20 (*m*, 40 H); 6,30 (*s*, H-C(12)); 5,34 (*d*, *J* = 3,7 Hz, H-C(6)); 5,10 (*d*, *J* = 10,6 Hz), 4,93 (*d*, *J* = 10,6 Hz), 4,84 (*d*, *J* = 10,6 Hz), 4,81-4,50 (*m*, 16 PhC*H*); 3,89-3,71 (*m*, 8 H); 3,67 (*t*, *J* = 8,7 Hz, H-C(3'')); 3,55 (*d*, *J* = 10,0 Hz, H'-C(6')); 3,43-3,40 (*m*, H-C(5")); 3,38 (*dd*, *J*= 7,5 Hz, 8,7 Hz, H-C(2")); 3,12 (*q*, *J* = 6,8 Hz, H-C(14)); 2,22 (*dd*, *J* = 3,9 Hz, 12,9 Hz, H-C(7)); 1,98 (*ddd*, *J* = 4,0 Hz, 13,1 Hz, 14,2 Hz, H'-C(7)); 1,85 (*dd, J* = 3,7 Hz, 14,3 Hz, H-C(8)); 1,44 (*d*, *J* = 6,8 Hz, Me-C(14)); 1,17 (*s*, *t*-Bu).

### 3,10-O-(2,3,4,6-tétra-O-benzyl-β-D-glucopyranosyl)ginkgolide B (12) :

IR (CHCl₃) : 3492, 3090, 3067, 3008, 2873, 1951, 1793, 1606, 1497, 1454, 1406, 1361, 1326, 1314, 1279, 1152, 1072, 1028, 988, 968, 903.

RMN ¹H (300 MHz, acétone (D₆)) : 7,46-7,24 (*m*, 40 H); 6,17 (*s*, H-C(12)); 5,30 (*s*, H-C(6)); 5,22 (*d*, *J* = 7,2 Hz, H-C(2)); 5,05 (*d*, *J* = 11,5 Hz), 4,93 (*d*, *J* = 11,2 Hz), 4,81-4,64 (*m*), 4,60 (*d*, *J* = 12,1 Hz), 4,51 (*d, J* = 11,8 Hz, 16 PhC*H*); 3,89 (*t*, *J* = 8,4 Hz, H-C(3')); 3,86-3,63 (*m*, irradiation à 5,27 → *d*, *J* = 9,0 Hz, H-C(2'), 8 H); 3,43-3,40 (*m*, H-C(5")); 3,40 (*dd*, *J* = 7,8 Hz, 8,7 Hz, H-C(2")); 3,12 (*q*, *J* = 6,8, H-C(14)); 2,02-1,80 (*m*, H-C(8), H-C(7), H'-C(7)); 1,44 (*d*, *J* = 6,5 Hz, Me-C(14)); 1,05 (*s*, *t*-Bu).

### 1-O-(2,3,4,6-tétra-O-benzyl-α-D-glucopyranosyl)-3-O-(2,3,4,6-tétra-O-benzyl-β-D-glucopyranosyl)ginkgolide B (13) :

IR (CHCl₃) : 3392, 3090, 3066, 3008, 2917, 2874, 1952, 1795, 1605, 1497, 1454, 1406, 1360, 1325, 1313, 1284, 1154, 1070, 1028, 986, 961, 923.
RMN ¹H (300 MHz, acétone (D₆)) : 7,45-7,22 (*m*, 40 H); 6,13 (*s*, H-C(12)); 5,58 (*m*, H-C(6)); 4,98 (*d*, *J* = 11,2 Hz), 4.96-4,75 (*m*, 9 PhC*H*); 4,65-4,58 (*m*, irradiation à 5,28 → *m*, H-C(1), 6 PhC*H*); 4,48 (*d*, *J* = 11,5 Hz, PhC*H*); 4,21 (*dt*, H-C(5')); 3,98 (*t*, *J* = 9,6 Hz, H-C(3')); 3,72-3,67 (*m*, 5 H); 3,62 (*dd*, *J* = 3,7 Hz, 9,6 Hz, H-C(2')); 3,58 (*t*, *J* = 9,0 Hz, H-C(4')); 3,47 (*t*, *J* = 9,0 Hz, irradiation à 3,35 → *d*, *J* = 9,3 Hz, H-C(3")); 3,35 (*dd*, *J* = 7,5 Hz, 9,0 Hz, H-C(2")); 3,15 (*q*, *J* = 7,0 Hz, H-C(14)); 3,04 (*dt*, H-C(5'')); 2,16-2,14 (*m*, H-C(7)); 1,93-1,84 (*m*, H'-C(7)); 1,39 (*d*, *J*= 6,9 Hz, Me-C(14));1,36-1,29 (*m*, H-C(8)); 1,13 (*s*, *t*-Bu).

### 1,3-O-(2,3,4,6-tétra-O-benzyl-β-D-glucopyranosyl)ginkgolide B (14) :

IR (CHCl₃) : 3462, 3090, 3066, 3008, 2873, 1952, 1796, 1702, 1604, 1497, 1454, 1406, 1361, 1322, 1278, 1177, 1148, 1082, 1028, 987, 956, 923, 905.
RMN ¹H (300 MHz, acétone (D₆)) : 7,44-7,16 (*m*, 40 H); 6,15 (*s*, H-C(12)); 5,43-5,42 (*m*, H-C(6)); 4,97 (*d*, *J* = 11,5 Hz), 4,90 (*d*, *J* = 10,9 Hz), 4,85 (*d*, *J* = 11,2 Hz), 4,83 (*d*, *J* = 11,2 Hz), 4,80-4,68 (*m*), 4,62 (*d*, *J* = 12,5 Hz), 4,61 (*d*, *J* = 12,1 Hz), 4,48 (*d*, *J* = 11,8 Hz, 16 PhC*H*); 3,93 (*d*, *J* = 2,2 Hz, H-C(6'), H'-C(6')); 3,83 (*dd*, *J* = 9,0 Hz, 9,7 Hz, H-C(4')); 3,74-3,63 (*m*, irradiation à 3,83 → *m*, H C(3'), 3 H); 3,55-3,46 (*m*, irradiation à 3,83 → *m*, H-C(5'), irradiation à 4,81 → *m*, H-C(2'), irradiation à 3,37 → *m*, H-C(3")); 3,37 (*dd*, *J* = 7,5 Hz, 9,0 Hz, irradiation à 4,65 → *d*, *J* = 9,0 Hz, H-C(2")); 3,25 (*m*, H-C(5"); 3,22 (*q*, *J* = 6,8 Hz, H-C(14)); 1,89 (*dd*, *J* = 6,5 Hz, 12,4 Hz, H-C(7)); 1,41 (*d*, *J* = 7,1 Hz, Me-C(14)); 1,09 (*s*, *t*-Bu).

### 3-O-(2,3,4,6-tétra-O-benzyl-α-D-glucopyranosyl)-10-O-(2,3,4,6-tétra-O-benzyl-β-D-glucopyranosyl)ginkgolide B (15) :

IR (CHCl₃) : 3534, 3090, 3066, 3008, 2926, 2874, 1951, 1795, 1706, 1603, 1497, 1454, 1404, 1361, 1324, 1313, 1261, 1139, 1072, 1028, 989, 966, 904.
RMN ¹H (300 MHz, acétone (D₆)) : 7,43-7,22 (*m*, 40 H); 6,19 (*s*, H-C(12)); 5,27 (*d*, *J* = 3,4 Hz, H-C(6)); 4,97 (*d*, *J* = 11,8 Hz), 4,93 (*d*, *J* = 11,8 Hz), 4,92 (*d*, *J* = 10,6 Hz), 4,87-4,73 (*m*), 4,68-4,48 (*m*, 16 PhC*H*); 3,99-3,94 (*m*, 1 H); 3,82-3,55 (*m*, 9 H); 3,39 (*dd*, *J* = 4,2 Hz, 7,3 Hz, irradiation à 4,80 → *m*, H-C(2')); 3,38 (*dd*, *J* = 6,5 Hz, 7,3 Hz, 1 H); 3,37 (*dd*, *J* = 7,8 Hz, 8,7 Hz, irradiation à 4,50 → *d*, *J* = 9,0 Hz, H-C(2")); 3,05 (*q*, *J* = 6,8 Hz, H-C(14)); 2,16-1,74 (*m*, 3 H); 1,41 (*d*, *J* = 6,8 Hz, Me-C(14)); 1,14 (*s*, *t*Bu).

### 1,3-O-(2,3,4,6-tétra-O-benzyl-β-D-glucopyranosyl)-10-O-(2,3,4,6-tétra-O-benzyl-α-D-glucopyranosyl)ginkgolide B (16) :

IR (CHCl₃) : 3089, 3066, 3008, 2926, 2872, 1951, 1791, 1709, 1604, 1497, 1454, 1363, 1312, 1279, 1073, 1028, 984, 910.
RMN ¹H (500 MHz, acétone (D₆)) : 7,45-7,01 (*m*, 60 H); 6,05 (*s*, H-C(12)); 5,44 (*d*, *J* = 3,6 Hz, H-C(6)); 5,26 (*d*, *J* = 10,7 Hz), 5,02 (*d*, *J* = 10,7 Hz), 4,99 (*d*, *J* = 11,1 Hz), 4,96 (*d*, *J* = 12,0 Hz), 4,95 (*d*, *J* = 10,6 Hz), 4,90 (*d*, *J* = 10,7 Hz), 4,83 (*d*, *J* = 11,4 Hz), 4,80 (*d*, *J* = 11,6 Hz), 4,78 (*d*, *J* = 11,4 Hz), 4,77 (*d*, *J* = 10,7 Hz), 4,69-4,59 (*m*), 4,48 (*d*, *J* = 12,6 Hz), 4,47 (*d*, *J* = 12,0 Hz), 4,45 (*d*, *J* = 11,3 Hz, 21 PhC*H*); 4,44 (*m*, H-C(3")); 4,41 (*d*, *J* = 12,1 Hz), 4,38 (*d*, *J* = 11,3 Hz), 4,31 (*d*, *J* = 12,6 Hz, 3 PhC*H*); 4,06 (*t*, *J* = 9,7 Hz, H-C(4')); 4,00 (*t*, *J* = 9,2 Hz, H-C(3")); 3,99 (*m*_{c}, H-C(6')); 3,94 (*dd*, *J* = 4,5 Hz, 10,6 Hz, H-C(2')); 3,73-3,63 (*m*, H-C(3'''), H-C(5'''), H-(6'''), H'-C(6'''), H-C(5'), H-C(4''')); 3,59-3,55 (*m*, H-C4"), H-C(6')); 3,45 (*dd*, *J* = 7,9 Hz, 9,4 Hz, H-C(2")); 3,40 (*t*, *J* = 7,7 Hz, H-C(2''')); 3,23 (*dd*, *J* = 3,7 Hz, 10,9 Hz, H-C(6")); 3,18 (*q*, *J* = 7,8 Hz, H-C(14)); 3,14 (*dd*, *J* = 1,6 Hz, 11.0 Hz, H'-C(6")), 2,95 (*m*_{c}, H-C(5")); 1,76 (*dd*, *J* = 4,1 Hz, 14,6 Hz, H-C(7)); 1,44 (*d*, *J =* 7,8 Hz, Me-C(14)); 1,33 (*m*, H-C(8)); 1,14 (*dt*, *J* = 4,0 Hz, 14,2 Hz, H'-C(7)); 1,04 (s, *t*-Bu).

### 1-O-(2,3,4,6-tétra-O-benzyl-α-D-glucopyranosyl)-3,10-O-(2,3,4,6-tétra-O-benzyl-β-D-glucopyranosyl)ginkgolide B (17) :

IR (CHCl₃) : 3090, 3066, 3008, 2913, 2872, 1951, 1794, 1605, 1497, 1454, 1399, 1360, 1328, 1310, 1279, 1145, 1074, 1028, 1007, 985, 902.
RMN ¹H (500 MHz, acétone (D₆)) : 7,42-7,05 (*m*, 60 H); 6,03 (*s*, H-C(12)); 5,36 (*d*, *J* = 3,7 Hz, H-C(6)); 5,17 (*d*, *J* = 10,6 Hz), 5,03 (*d*, *J* = 10,9 Hz), 4,99 (*d*, *J* = 11,7 Hz), 4,93 (*d*, *J* = 11,3 Hz), 4,88 (*d*, *J* = 11,2 Hz), 4,85 (*d*, *J* = 11,0 Hz), 4,80 (*d*, *J* = 12,5 Hz), 4,78 (*d*, *J* = 11,2 Hz), 4,74 (*d*, *J* = 11,2 Hz), 4,70 (*d*, *J* = 11,0 Hz), 4,66 (*d*, *J* = 11,9 Hz), 4,62 (*d*, *J* = 10,5 Hz), 4,60 (*d*, *J* = 11,0 Hz), 4,51 (*d*, *J* = 10,5 Hz), 4,50 (*d*, *J* = 11,7 Hz), 4,49 (*d*, *J* = 10,6 Hz), 4,47 (*m*), 4,43 (*d*, *J =* 12,1 Hz), 4,39 (*d*, *J* = 11,2 Hz), 4,37 (*d*, *J* = 12,0 Hz, 24 PhC*H*); 4,19-4,13 (*m*, H-C(5'), H-C(4'''), H-C(3')); 3,97 (*t*, *J* = 7,8 Hz, H-C(2")); 3,87 (*m*, H-C(4')); 3,84 (*t*, *J* = 7,8 Hz, H-C(3")); 3,82-3,67 (*m*, 7 H); 3,66 (*dd*, *J* = 4,4 Hz, 9,6 Hz, H-C(2')); 3,46 (*dd*, *J* = 2,9 Hz, 10,8 Hz, 1 H); 3.40-3,38 (*m*, 1H); 3,36 (*dd*, *J* = 7,6 Hz, 8,9 Hz, H-C(2''')); 3,10 (*q*, *J* = 7,5 Hz, H-C(14)); 3,07 (*m*, 1 H); 1,94 (*dt*, *J* = 4,0 Hz, 14,1 Hz, H-C(7)); 1,75 (*dd*, *J* = 4,0 Hz, 14,4 Hz, H'-C(7)); 1,36 (*dd*, *J* = 4,1 Hz, 13,4 Hz, H-(8)); 1,30 (*d*, *J* = 7,5 Hz, Me-C(14)); 0,94 (*s*, *t*-Bu).

### 1,3,10-O-(2,3,4,6-tétra-O-benzyl-β-D-glucopyranosyl)ginkgolide B (18) :

IR (CHCl₃) : 3090, 3066, 3008, 2913, 2872, 1951, 1792, 1702, 1654, 1606, 1586, 1497, 1454, 1400, 1360, 1314, 1280, 1148, 1074, 1028, 987, 905.
RMN ¹H (300 MHz, acétone (D₆)) : 7,50-7,09 (*m*, 60 H); 6,11 (*s*, H-C(12)); 5.66 (*s*, H-C(6)); 5,10 (*d*, *J* = 11,5 Hz), 4,99 (*d*, *J* = 11,2 Hz), 4,97 (*d*, *J* = 11,2 Hz), 4,88 (*d, J =* 11,2 Hz), 4,86 (*d*, *J* = 11,2 Hz), 4,77 (*d*, *J* = 11,2 Hz), 4,78-4,53 (*m*), 4,47 (*d*, *J* = 11,8 Hz), 4,43 (*d*, *J* = 11,2 Hz, 24 PhC*H*); 4,10 (*t*, *J* = 8,1 Hz, irradiation à 5,01 → *d*, *J* = 8,4 Hz, H-C(2")); 4,00-3,94 (*m*, irradiation à 3,42 → *m*, H-C(3'''), 1 H); 3,86-3,66 (*m*, irradiation à 4,10 → *m*, II-C(3"), 9 II), 3,58 (*dd*, *J* = 7,9 Hz, 9,2 Hz, irradiation a 5,34 → *d*, *J* = 9,0 Hz, H-C(2')); 3,57 (*t*, *J* = 9,0 Hz, 1 H); 3,46-3,39 (*m*, irradiation à 4,83 → *m*, H-C(2'''), 2 H); 3,28 (*q*, *J* = 7,2 Hz, H-C(14)); 1,91-1,85 (*m*, 1 H); 1,45 (*d*, *J* = 7,5 Hz, Me-C(14)); 0,98 (*s*, *t*Bu).

### 2.5. 1-O-(β-D-glucopyranosyl)ginkgolide B (19) :

Un mélange de **7** (73,0 mg, 0,077 mmol), tel qu'obtenu par exemple dans l'étape 2.1. ou 2.2., et de Pd/C 10 % (100 mg) dans MeOH (3,0 ml) a été hydrogéné durant 17 heures sous une pression de 3,5 atm. Après filtration et évaporation, on a obtenu **19** (42,7 mg, 94 %).
RMN ¹H (400 MHz, acétone(D₆)) : 6,09 (*s*, H-C(12)); 5,55-5,35 (*m*, H-C(6)); 3,85-3,79 (*m*, H-C(6')); 3,66 (*dd*, *J* = 5,4 Hz, 11,6, H'-C(6')); 3,47 (*t*, *J* = 8,8 Hz, H-C(3')); 3,40-3,33 (*m*, H-C(4'), H-C(5')); 3,28 (*dd*, *J* = 7,8 Hz, 8,9 Hz, H-C(2')); 3,09 (*q*, *J* = 7,2 Hz, H-C(14)); 2,25-2,18 (*m*, H-C(7), H'-C(7)); 1,94 (*dd*, *J* = 6,5 Hz, 12,4 Hz, H-C(8)); 1,25 (*d*, *J* = 7,4 Hz, Me-C(14)); 1,26 (*s*, *t*-Bu).
RMN ¹³C (100 MHz, acétone (D₆)) : 176,4 (*s*); 173,9 (*s*); 170,9 (*s*); 110,6 (*d*); 100,8 (*s*); 94,0 (*d*); 85,5 (*s*); 83,2 (*d*); 80,1 (*d*); 77,8 (2*d*); 75,0 (*d*); 73,2 (*s*); 71,5 (*d*); 70,4 (*d*); 69,6 (*s*); 62,8 (*t*); 50,0 (*d*); 42,4 (*d*); 37,7 (*t*); 33,1 (*s*); 31,9 (3*q*); 8,8 (*q*).

### 2.6. 1-O-(α-D-glucopyranosyl)ginkgolide B (20) :

Un mélange de **8** (20,1 mg, 0,021 mmol), tel qu'obtenu par exemple dans l'étape 2.1., et de Pd/C 10 % (50 mg) dans MeOH (3,0 ml) a été hydrogéné durant 17 heures sous une pression de 2,1 atm. Après filtration et évaporation, on a obtenu **20** (6,5 mg, 52 %).
RMN ¹H (300 MHz, CD₃OD) : 6,00 (*s*, H-C(12)); 5,67 (*d*, *J* = 3,1 Hz, H-C(6)); 3,90 (*ddd*, *J* = 2,6 Hz, 3,4 Hz, 9,6 Hz, H-C(5')); 3,81 (*dd*, *J* = 2,5 Hz, 12,1 Hz, H-C(6')); 3,73 (*dd*, *J* = 3,7 Hz, 11,8 Hz, H'-C(6')); 3,61 (*dd*, *J* = 9,0 Hz, 9,9 Hz, H-C(3')); 3,47 (*dd*, *J* = 3,7 Hz, 9,9 Hz, irradiation à 5,11 → *d*, *J* = 9,7 Hz, H-C(2')); 3,44 (*dd*, *J* = 9,0 Hz, 9,9 Hz, H-C(4')); 3,07 (*q*, *J* = 7,2 Hz, H-C(14)); 2,22-2,10 (*m*, H-C(7), H'-C(7)); 1,89 (*dd*, *J* = 5,6 Hz, 12,5 Hz, H-C(8)); 1,24 (*d*, *J* = 7,5 Hz, Me-C(14)); 1,11 (*s*, *t*-Bu).
RMN ¹³C (100 MHz, CD₃OD) : 178,1 (*s*); 175,2 (*s*); 173,1 (*s*); 110,8 (*d*); 103,2 (*s*); 94,2 (*d*); 85,8 (*s*); 81,1 (*d*); 80,3 (*d*); 74,9 (*s*); 74,7 (*d*); 74,2 (*d*); 73,3 (*d*); 71,0 (*d*); 70,6 (*s*); 70,4 (*d*); 61,9 (*t*); 50,8 (*d*); 42,8 (*d*); 37,8 (*t*); 33,4 (*s*); 29,6 (3*q*); 9,6 (*q*).

### 2.7. 10-O-(β-D-glucopyranosyl)ginkgolide B (21) :

Un mélange de **9** (32,0 mg, 0,034 mmol), tel qu'obtenu par exemple dans l'étape 2.1. ou 2.2., et de Pd/C 10 % (100 mg) dans MeOH (3,0 ml) a été hydrogéné durant 17 heures sous une pression de 3,5 atm. Après filtration, évaporation et chromatographie éclair (750 g de silice, éluant AcOEt/MeOH/H₂O 8,5:1,5:1), on a obtenu **21** (19,8 mg, 88 %).
RMN ¹H (500 MHz, acétone (D₆)) : 6,15 (*s*, H-C(12)); 5,43 (*d*, *J* = 4,0 Hz, H-C(6)); 5,27 (*s*, échangé par D₂O, HO-C(3)); 3,87-3,83 (*m*, H-C(6')); 3,78 (*m*, H'-C(6')); 3,66-3,62 (*m*, H-C(5')); 3,49-3,45 (*m*, H-C(2-)); 3,41-3,34 (*m*, H-C(3'), H-C(4')); 3,02 (*q*, *J* = 7,1 Hz, H-C(14)); 2,28 (*dd*, *J* = 4,3 Hz, 13,5 Hz, H-C(7)); 2,08-2,01 (*m*, H'-C(7)); 1,93 (*dd*, *J* = 4,3 Hz, 14,6 Hz, H-C(8)); 1,26 (*d*, *J* = 7,1 Hz, Me-C(14)); 1,21 (*s*, *t*-Bu).
RMN ¹³C (125 MHz, acétone (D₆)) : 176,7 (*s*); 171,6 (*s*); 171,1 (*s*); 110,0 (*d*); 99,8 (*s*); 93,3 (*d*); 84,1 (*s*); 79,4 (*d*); 78,8 (*d*); 77,7 (*d*); 75,6 (*d*); 75,5 (*d*); 73,6 (*s*); 72,7 (*d*); 71,6 (*d*); 69,2 (*s*); 62,6 (*t*); 50,5 (*d*); 43,0 (*d*); 37,7 (*t*); 32,9 (*s*); 29,7 (3*q*); 8,2 (*q*).

### 2.8. 10-O-(α-D-glucopyranosyl)ginkgolide B (22) :

Un mélange de **10** (45,1 mg, 0,048 mmol), tel qu'obtenu par exemple dans l'étape 2.1. ou 2.2., et de Pd/C 10 % (50 mg) dans MeOH (3,0 ml) a été hydrogéné durant 17 heures sous une pression de 2,1 atm. Après filtration et évaporation, on a obtenu **22** (27,5 mg, 98 %).
RMN ¹H (300 MHz, CD₃OD) : 6,14 (*s*, H-C(12)); 5,44 (*d*, *J* = 4,0 Hz, H-C(6)); 3,79-3,73 (*m*, H-C(5')); 3,67-3,44 (*m*, 5 H); 3,06 (*q*, *J* = 7,2 Hz, H-C(14)); 2,23 (*dd*, *J* = 4,4 Hz, 13,4 Hz, H-C(7)); 2,01 (*ddd*, *J* = 4,1 Hz, 13,6 Hz, 14,3 Hz, H'-C(7)); 1,81 (*dd*, *J* = 4,3 Hz, 14,3 Hz, H-C(8)); 1,21 (*d*, *J* = 7,2 Hz, Me-C(14)); 1,12 (*s*, *t*-Bu).
RMN ¹³C (100 MHz, CD₃OD) : 178,5 (*s*); 173,1 (*s*); 172,8 (*s*); 111,2 (*d*); 100,3 (*s*), 94,7 (*d*), 83,6 (*s*); 80,4 (*d*); 74,8 (*d*); 74,8 (*d*); 74,1 (*d*); 73,5 (*d*); 73,4 (*s*); 72,4 (*d*); 70,2 (*d*); 69,8 (*s*); 61,5 (*t*); 50,2 (*d*); 43,4 (*d*); 38,3 (*t*); 33,4 (*s*); 29,9 (3*q*); 8,1 (*q*).

### 2.9. 3-O-(β-D-glucopyranosyl)-10-O-(α-D-glucopyranosyl)ginkgolide B (23) :

Un mélange de **11** (36,1 mg, 0,025 mmol), tel qu'obtenu par exemple dans l'étape 2.2. ou 2.3., et de Pd/C 10 % (50 mg) dans MeOH (3,0 ml) et de l'acétone (1,5 ml) a été hydrogéné durant 21 heures sous une pression de 1,2 atm. Après filtration et évaporation, on a obtenu **23** (18,3 mg, 99 %).
RMN ¹H (300 MHz, CD₃OD) : 6,17 (*s*, H-C(12)); 5,50 (*d*, *J* = 4,1 Hz, H-C(6)); 3,78-3,15 (*m*, 12 H); 3,08 (*q*, *J* = 6,8 Hz, H-C(14)); 2,24 (*dd*, *J* = 4,4 Hz, 13,7 Hz, H-C(7)); 2,02 (*ddd*, *J* = 4,0 Hz, 13,7 Hz, 14,0 Hz, H'-C(7)); 1,80 (*dd*, *J* = 4,0 Hz, 14,3 Hz, H-C(8)); 1,31 (*d*, *J*= 6,8 Hz, Me-C(14)); 1,12 (*s*, *t*-Bu).

### 2.10. 3,10-O-(β-D-glucopyranosyl)ginkgolide B (24) :

Un mélange de **12** (37,9 mg, 0,026 mmol), tel qu'obtenu par exemple dans l'étape 2.2., et de Pd/C 10 % (50 mg) dans MeOH (3,0 ml) et de l'acétone (1,0 ml) a été hydrogéné durant 21 heures sous une pression de 2,3 atm. Après filtration et évaporation, on a obtenu **24** (18,3 mg, 95 %).
RMN ¹H (300 MHz, CD₃OD): 6,15 (*s*, H-C(12)); 5,52 (*d*, *J* = 4,0 Hz, H-C(6)); 3,88 (*dd, J* = 1,6 Hz, 12,5 Hz, 1 H); 3,75 (*dd*, *J* = 2,3 Hz, 12,5 Hz, 1 H); 3,67 (*dd*, *J=* 5,8 Hz, 12,5 Hz, 1 H); 3,61 (*dd*, *J* = 4,7 Hz, 12,2 Hz, 1 H); 3,43-3,14 (*m*, 8H); 3.02 (*q*, *J* = 6,8 Hz, H-C(14)); 2,27 (*dd, J* = 4,4 Hz, 13,4 Hz, H-C(7)); 2,03 (*ddd*, *J* = 4,4 Hz, 14,0 Hz, 14,6 Hz, H'-C(7)); 1,89 (*dd*, *J* = 4,0 Hz, 14,6 Hz, H-C(8)); 1,33 (*d*, *J =* 6,8 Hz, Me-C(14)); 1,13 (*s*, *t*-Bu).
RMN ¹³C (100 MHz, CD₃OD): 178,9 (*s*); 172,7 (*s*); 172,1 (*s*); 111,8 (*d*); 100,5 (*s*); 90,0 (*s*); 88,1 (*d*); 80,3 (*d*); 79,2 (*d*); 78,6 (*d*); 77,9 (*d*); 77,8 (*d*); 76,6 (*d*); 75,5 (*d*); 74,6 (*d*); 74,0 (*s*); 73,5 (*d*); 71,3 (*d*); 70,9 (*d*); 69,6 (*s*); 62,6 (*t*); 62,3 (*t*); 51,2 (*d*); 43,9 (*d*); 38,0 (*t*); 33,2 (*s*); 29,7 (3*q*); 8,3 (*q*).

### 2.11. 1,3-O-(β-D-glucopyranosyl)ginkgolide B (25) :

Un mélange de **14** (17,1 mg, 0,012 mmol), tel qu'obtenu par exemple dans l'étape 2.2., et de Pd/C 10 % (50 mg) dans MeOH (3,0 ml) et de l'acétone (1,5 ml) a été hydrogéné durant 21 heures sous une pression de 1,2 atm. Après filtration et évaporation, on a obtenu **25** (8,5 mg, 97 %).
RMN ¹H (300 MHz, CD₃OD) : 6,08 (*s*, H-C(12)); 5,58 (*d*, *J* = 2,5 Hz, H-C(6)); 3,86 (*dd*, *J* = 2,5 Hz, 12,1 Hz, 1 H); 3,78 (*dd*, *J* = 3,3 Hz, 13,1 Hz, 1 H); 3,72 (*dd*, *J* = 5,0Hz, 12,1 Hz, 1 H); 3,59 (*dd, J* = 5,3 Hz, 12,1 Hz, 1 H); 3,41-3,18 (*m*, 8H); 3,11 (*q*, *J* = 7,2 Hz, H-C(14)); 2,24-2,10 (*m*, H-C(7), H'-C(7)); 1,89 (*dd*, *J* = 5,3 Hz, 13,1 Hz, H-C(8)); 1,32 (*d*, *J* = 7,2 Hz, Me-C(14)); 1,12 (*s*, *t*-Bu).
RMN ¹³C (100 MHz, CD₃OD) : 178,5 (*s*); 175,1 (*s*); 172,0 (*s*); 111,4 (*d*); 101,4 (*s*); 91,3 (*s*); 88,9 (*d*); 84,5 (*d*); 81,0 (*d*); 78,5 (*d*); 78,0 (*d*); 77,9 (*d*); 77,8 (*d*); 75,2 (*d*); 74,6 (*d*); 73,4 (s); 71,2 (*d*); 71,1 (*d*); 70,4 (*s*); 70,0 (*d*); 62,4 (*t*); 62,3 (*t*); 50,6 (*d*); 43,6 (*d*); 38,0 (*t*); 33,4 (*s*); 29,6 (3*q*); 9,2 (*q*).

### 2.12. 1,3,10-O-(β-D-glucopyranosyl)ginkgolide B (26) :

Un mélange de **16** (53,0 mg, 0,027 mmol), tel qu'obtenu par exemple dans l'étape 2.3., et de Pd/C 10 % (50 mg) dans MeOH (3,0 ml) et de l'acétone (1,5 ml) a été hydrogéné durant 21 heures sous une pression de 1,2 atm. Après filtration et évaporation, on a obtenu **26** (23,5 mg, 97 %).
RMN ¹H (300 MHz, CD₃OD) : 6,05 (*s*, H-C(12)); 5,75 (*d*, *J* = 3,4 Hz, H-C(6)); 3,90-3,16 (*m*, 18 H); 3,08 (*q*, *J* = 7,5 Hz, H-C(14)); 2,30 (*dt*, *J* = 4,1 Hz, 14,0 Hz, H-C(7)); 2,18-2,11 (*m*, H'-C(7)); 1,91 (*dd*, *J* = 4,4 Hz, 14,3 Hz, H-C(8)); 1,32 (*d*, *J* = 6,8 Hz, Me-C(14)); 1,14 (*s*, *t*-Bu).
RMN ¹³C (100 MHz, CD₃OD): 177,8 (*s*); 172,8 (*s*); 172,1 (*s*); 109,8 (*d*); 104,8 (*s*); 92,3 (*s*); 91,5 (*d*); 85,6 (*d*); 80,6 (*d*); 78,9 (*d*); 78,5 (*d*); 78,2 (*d*); 78,1 (*d*); 78,0 (*d*); 77,9 (*d*); 77,7 (*d*); 76,1 (*s*); 75,3 (*d*); 74,7 (*d*); 74,4 (*d*); 71,8 (*d*); 71,5 (*d*); 70,9 (*d*); 70,8 (*s*); 62,9 (*t*); 62,8 (*t*); 61,9 (*t*); 51,7 (*d*); 43,1 (*d*); 37,6 (*t*); 33,1 (*s*); 30,2 (3*q*); 11,9 (*q*).

### Exemple 3 : 3-O-[(4-O-β-D-galactopyranosyl)-β-D-glucopyranosyl]ginkgolide A (30) :

### 3.1. 10-O-acétyl-3-O-[2,3,6-tri-O-benzyl-4-O-(2,3,4,6-tétra-O-benzyl-β-D-galactopyranosyl)-β-D-glucopyranosyl]ginkgolide A (28) :

Du ginkgolide A (**1**) (7 mg, 15 µmol) a été traité avec une solution de 1-azi-2,3,6-tri-O-benzyl-4-O-[2,3,4,6-tétra-O-benzyl-D-galactopyranosyl] 1-déoxy-D-glucopyranose (**27**) (50 mg, 51 µmol ; obtenu selon des méthodes semblables à celles citées pour les autres glycosyl diazirines) dans du THF (1 ml), et agité pendant 1 heure à 25° C. Après évaporation et HPLC (éluant hexane/acétone 3:2), on a obtenu **28** (20 mg, 90 %).

RMN ¹H (300 MHz, CDCl₃) : 7,41-7,03 (*m*, 35 H); 6,10(*s*); 6,05 (*s*, H-C(10), H-C(12)); 5,16-5,08 (*m*, 2H); 4,95 (*d*, *J* = 11,2 Hz, PhCH); 4,83-4,33 (*m,* 15 H); 4,25 (*d*, *J* = 12,0 Hz, PhCH); 4,10 (*t*, *J* = 9,1 Hz, 1 H); 3,90-3,34 (*m*, 10 H); 3,17-3,06 (*m*, 2H); 2,70 (*dd*, *J* = 7,5 Hz, 14,9 Hz, H-C(1)); 2,21 (*s*, AcO); 2,15-1,73 (*m*, 4 H); 1,45 (*d*, *J* = 6,6 Hz, Me-C(14)); 1,04 (*s*, *t*-Bu).

### 3.2. 10-O-acétyl-3-O-[4-O-(β-D-galactopyranosyl)-β-D-glucopyranosyl]ginkgolide A (29) :

**28** (20 mg, 14 µmol) en présence de Pd/C 10 % (100 mg) dans MeOH (5ml) a été hydrogéné sous une pression de 3 atm durant 17 heures. Après filtration et évaporation, on a obtenu **29** (11 mg, 100 %).
RMN ¹H (300 MHz, CD₃OD) : 6,24 (*s*); 6,17 (*d*, H-C(10), H-C(12)); 5,53 (*t*, *J* = 8,4 Hz, H-C(2)); 4,98 (*d*, *J* = 3,7 Hz, H-C(6)); 4,53 (*d*, *J* = 7,5 Hz, H-C(1')); 4,36 (*d*, *J* = 7,2 Hz, H-C(1"); 3,81-3,24 (*m*, 12 H); 3,15 (*q*, *J* = 6,8 Hz, H-C(14); 3,03 (*dd*, *J* = 7,8 Hz, 14,6 Hz, H-C(1)); 2,26-2,21 (*m*, 1 H); 2,21 (*s*, AcO); 2,09 (*ddd*, *J* = 4,0 Hz, 13,7 Hz, 13,8 Hz, H-C(7)); 1,98-1,86 (*m*, 2H); 1,35 (*d*, *J* = 6,8 Hz, Me-C(14)); 1,05 (*s*, *t*-Bu).

### 3.3. 3-O-[4-O-(β-D-galactopyranosyl)-β-D-glucopyranosyl]ginkgolide A (30) :

**30** est obtenu par désacétylation de **29** par des méthodes classiques (traitement par exemple avec de l'ammoniaque dans du méthanol; cf. étape 1.3.).

## Revendications

1. Produit **caractérisé en ce qu'**il répond à la formule générale (**I**) 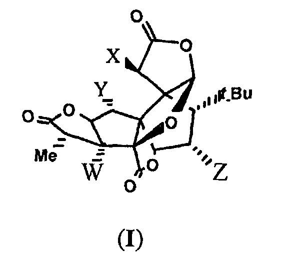 dans laquelle W, X, Y et Z représentent indépendamment les radicaux H, OH, alkoxy linéaire ou ramifié ou O-G_{S}, G_{S}-OH représentant un mono- ou un disaccharide, ou un de leurs dérivés ou analogues,
étant entendu que l'un au moins de W, X, Y ou Z représente un radical O-G_{S}.

2. Produit selon la revendication 1, **caractérisé en ce que** X représente un radical OH ou O-G_{S}, G_{S}-OH représentant un mono- ou un disaccharide, ou un de leurs dérivés ou analogues, et :
- ou bien W représente un radical OH ou O-G_{S}, Y représente H et Z représente H ;
- ou bien W représente un radical OH ou O-G_{S}, Y représente un radical OH ou O-G_{S} et Z représente H ;
- ou bien W représente un radical OH ou O-G_{S}, Y représente un radical OH ou O-G_{S} et Z représente un radical OH ou O-G_{S} ;
- ou bien W représente un radical OH ou O-G_{S}, Y représente H et Z représente un radical OH ou O-G_{S} ;
- ou bien W représente H, Y représente un radical OH ou O-G_{S} et Z représente un radical OH ou O-G_{S} ;
- ou bien W représente un radical OH ou O-G_{S}, Y représente un radical alkoxy linéaire ou ramifié et Z représente H ;
étant entendu que l'un au moins de W, X, Y ou Z représente un radical O-G_{S}.

3. Produit selon la revendication 1 ou 2, **caractérisé en ce que** X représente un radical OH ou O-G_{S}, G_{S}-OH représentant un mono- ou un disaccharide, ou un de leurs dérivés ou analogues, et :
- ou bien W représente un radical OH ou O-G_{S}, Y représente H et Z représente H ;
- ou bien W représente un radical OH ou O-G_{S}, Y représente un radical OH ou O-G_{S} et Z représente H ;
- ou bien W représente un radical OH ou O-G_{S}, Y représente un radical alkoxy linéaire ou ramifié et Z représente H ;
étant entendu que l'un au moins de W, X, Y ou Z représente un radical O-G_{S}.

4. Produit selon la revendication 1, 2 ou 3, **caractérisé en ce que** G_{S} est choisi de telle sorte que G_{S}-OH appartienne au groupe composé de l'abéquose, du rhamnose, de l'arabinose, du ribose, du xylose, du 2-déoxy-ribose, du glucose, du galactose, du mannose, du 2-déoxyglucose, du fructose, du fucose, de la N-acétylglucosamine, de la N-acétylallosamine, de la galactosamine, de la mannosamine, du saccharose, du lactose, du maltose, du cellobiose et du tréhalose.

5. Produit selon l'une quelconque des revendications 1 à 4, **caractérisé en ce que** G_{S} est choisi de telle sorte que G_{S}-OH appartienne au groupe composé du glucose et du lactose.

6. Produit selon la revendication 1, **caractérisé en ce qu'**il est choisi parmi :
- le 3-O-(β-D-glucopyranosyl)ginkgolide A ;
- le 1-O-(β-D-glucopyranosyl)ginkgolide B ;
- le 1-O-(α-D-glucopyranosyl)ginkgolide B ;
- le 10-O-(β-D-glucopyranosyl)ginkgolide B ;
- le 10-O-(α-D-glucopyranosyl)ginkgolide B ;
- le 3-O-(β-D-glucopyranosyl)-10-O-(α-D-glucopyranosyl)ginkgolide B ;
- le 3,10-O-(β-D-glucopyranosyl)ginkgolide B ;
- le 1,3-O-(β-D-glucopyranosyl)ginkgolide B ;
- le 1,3,10-O-(β-D-glucopyranosyl)ginkgolide B ; et
- le 3-O-[4-O-(β-D-galactopyranosyl)-β-D-glucopyranosyl]ginkgolide A.

7. A titre de médicament, un produit selon l'une quelconque des revendications 1 à 6.

8. Composition pharmaceutique comprenant, à titre de principe actif, au moins un produit selon la revendication 7.

9. Utilisation d'un produit selon l'une des revendications 1 à 6 pour fabriquer un médicament destiné à traiter les maladies vasculaires, en particulier les maladies cardiovasculaires.

10. Procédé de préparation d'un produit selon l'une des revendications 1 à 6, **caractérisé en ce qu'**il comprend principalement une étape de glycosylation, laquelle consiste en une réaction d'un composé de formule générale (**III**) représentée ci-dessous, 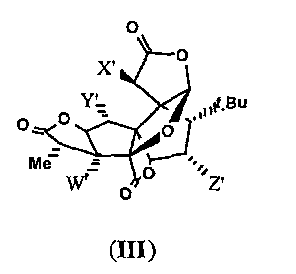 dans laquelle W', X', Y' et Z' représentent indépendamment un radical H, OH, alkoxy linéaire ou ramifié ou O-G_{X}, G_{X} étant un groupe protecteur d'un groupe hydroxy pouvant de préférence être éliminé en milieu neutre ou basique, étant entendu que l'un au moins de W', X', Y' et Z' représente OH,
avec une glycosyl diazirine de formule générale (**II**) représentée ci-dessous, 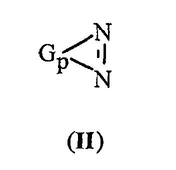 ledit procédé pouvant également comprendre une ou plusieurs étapes de protection et/ou déprotection de groupes hydroxy, et le composé (**II**) étant une diazirine dérivée d'un sucre Gₚ-OH dont tous les groupes hydroxy sauf celui porté par le carbone anomérique ont été protégés, par exemple par des radicaux benzyle ou silyle, le groupe hydroxy en position anomérique et l'hydrogène porté par le même atome de carbone ayant été substitués par un groupe azi.

## Patentansprüche

1. Produkt, **dadurch gekennzeichnet, daß** es der allgemeinen Formel (**I**) entspricht 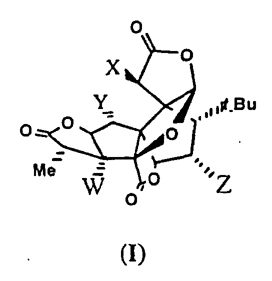 in der W, X, Y und Z unabhängig voneinander die Reste H, OH, verzweigtes oder unverzweigtes Alkoxy oder O-G_{S} darstellen, wobei G_{S}-OH ein Mono- oder ein Disaccharid oder eines ihrer Derivate oder Analoge darstellt,
wobei gilt, daß mindestens eines der Symbole W, X, Y oder Z einen O-G_{S}-Rest darstellt.

2. Produkt nach Anspruch 1, **dadurch gekennzeichnet, daß** X einen der Reste OH oder O-G_{S} darstellt, wobei G_{S}-OH ein Mono- oder ein Disaccharid oder eines ihrer Derivate oder Analoge darstellt, und
- entweder W einen der Reste OH oder O-G_{S} darstellt, Y H darstellt und Z H darstellt;
- oder W einen der Reste OH oder O-G_{S} darstellt, Y einen der Reste OH oder O-G_{S} darstellt und Z H darstellt;
- oder W einen der Reste OH oder O-G_{S} darstellt, Y einen der Reste OH oder O-G_{S} darstellt und Z einen der Reste OH oder O-G_{S} darstellt;
- oder W einen der Reste OH oder O-G_{S} darstellt, Y H darstellt und Z einen der Reste OH oder O-G_{S} darstellt;
- oder W H darstellt, Y einen der Reste OH oder O-G_{S} darstellt und Z einen der Reste OH oder O-G_{S} darstellt;
- oder W einen der Reste OH oder O-G_{S} darstellt, Y einen verzweigten oder unverzweigten Alkoxyrest darstellt und Z H darstellt;
wobei gilt, daß mindestens eines der Symbole W, X, Y oder Z einen O-G_{S}-Rest darstellt.

3. Produkt nach Anspruch 1 oder 2, **dadurch gekennzeichnet, daß** X einen der Reste OH oder O-G_{S} darstellt, wobei G_{S}-OH ein Mono- oder ein Disaccharid oder eines ihrer Derivate oder Analoge darstellt, und
- entweder W einen der Reste OH oder O-G_{S} darstellt, Y H darstellt und Z H darstellt;
- oder W einen der Reste OH oder O-G_{S} darstellt, Y einen der Reste OH oder O-G_{S} darstellt und Z H darstellt;
- oder W einen der Reste OH oder O-G_{S} darstellt, Y einen verzweigten oder unverzweigten Alkoxyrest darstellt und Z H darstellt;
wobei gilt, daß mindestens eines der Symbole W, X, Y oder Z einen O-G_{S}-Rest darstellt.

4. Produkt nach Anspruch 1, 2 oder 3, **dadurch gekennzeichnet, daß** Gs so gewählt ist, daß G_{S}-OH zu der Gruppe gehört, die aus den folgenden Stoffen besteht: Abequose, Rhamnose, Arabinose, Ribose, Xylose, 2-Deoxyribose, Glucose, Galactose, Mannose, 2-Deoxyglucose, Fructose, Fucose, N-Acetylglucosamin, N-Acetylallosamin, Galactosamin, Mannosamin, Saccharose, Lactose, Maltose, Cellobiose und Trehalose.

5. Produkt nach einem der Ansprüche 1 bis 4, **dadurch gekennzeichnet, daß** G_{S} so gewählt ist, daß G_{S}-OH zu der Gruppe gehört, die aus Glucose und Lactose besteht.

6. Produkt nach Anspruch 1, **dadurch gekennzeichnet, daß** es ausgewählt ist aus:
- 3-O-(β-D-Glucopyranosyl)ginkgolid A;
- 1-O-(β-D-Glucopyranosyl)ginkgolid B;
- 1-O-(α-D-Glucopyranosyl)ginkgolid B;
- 10-O-(β-D-Glucopyranosyl)ginkgolid B;
- 10-O-(α-D-Glucopyranosyl)ginkgolid B;
- 3-O-(β-D-Glucopyranosyl)-10-O-(α-D-Glucopyranosyl) ginkgolid B;
- 3,10-O-(β-D-Glucopyranosyl)ginkgolid B;
- 1,3-O-(β-D-Glucopyranosyl)ginkgolid B;
- 1,3,10-O-(β-D-Glucopyranosyl)ginkgolid B; und
- 3-O-[4-O-(β-D-Galactopyranosyl)-β-D-glucopyranosyl] ginkgolid A;

7. Ein Produkt nach einem der Ansprüche 1 bis 6 in Form eines Medikaments.

8. Pharmazeutische Zusammensetzung, die als Wirkstoff mindestens ein Produkt nach Anspruch 7 enthält.

9. Verwendung eines Produkts nach einem der Ansprüche 1 bis 6 zur Herstellung eines Medikaments, das für die Behandlung von vaskulären Krankheiten, insbesondere von kardiovaskulären Krankheiten, bestimmt ist.

10. Verfahren zur Herstellung eines Produkts nach einem der Ansprüche 1 bis 6, **dadurch gekennzeichnet, daß** es hauptsächlich einen Schritt der Glykosylierung umfaßt, der in einer Reaktion einer Verbindung der unten dargestellten allgemeinen Formel (**III**) 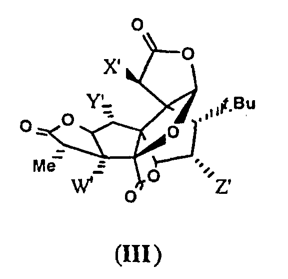 in der W', X', Y' und Z' unabhängig voneinander einen der Reste H, OH, verzweigtes oder unverzweigtes Alkoxy oder O-G_{X} darstellen, wobei G_{X} eine Schutzgruppe einer Hydroxygruppe ist, die vorzugsweise in neutralem oder basischem Medium entfernt werden kann, wobei gilt, daß mindestens eines der Symbole W', X', Y' und Z' OH darstellt,
mit einem Glykosyldiazirin der unten dargestellten allgemeinen Formel (**II**) besteht 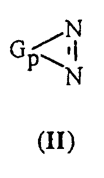 wobei dieses Verfahren auch einen oder mehrere Schritte des Schützens und/oder Entschützens von Hydroxygruppen umfassen kann und die Verbindung (**II**) ein Diazirin ist, das von einem Zucker Gₚ-OH abgeleitet ist, von dem alle Hydroxygruppen, mit Ausnahme der von dem anomerischen Kohlenstoff getragenen Gruppe, beispielsweise durch Benzyl- oder Silylreste geschützt wurden, wobei die Hydroxygruppe in anomerischer Stellung und der von demselben Kohlenstoffatom getragene Wasserstoff durch eine Azigruppe substituiert wurden.

## Claims

1. Product **characterized in that** it corresponds to general formula (**I**) 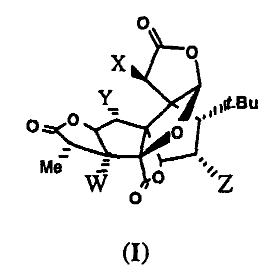 in which W, X, Y and Z independently represent the H, OH, linear or branched alkoxy or O-G_{S} radicals, G_{S}-OH representing a mono- or a disaccharide, or one of their derivatives or analogues,
it being understood that at least one of W, X, Y or Z represents an O-G_{S} radical.

2. Product according to claim 1, **characterized in that** X represents an OH or O-G_{S} radical, G_{S}-OH representing a mono- or a disaccharide, or one of their derivatives or analogues, and:
- either W represents an OH or O-G_{S} radical, Y represents H and Z represents H;
- or W represents an OH or O-G_{S} radical, Y represents an OH or O-G_{S} radical and Z represents H;
- or W represents an OH or O-G_{S} radical, Y represents an OH or O-G_{S} radical and Z represents an OH or O-G_{S} radical;
- or W represents an OH or O-G_{S} radical, Y represents H and Z represents an OH or O-G_{S} radical;
- or W represents H, Y represents an OH or O-G_{S} radical and Z represents an OH or O-G_{S} radical;
- or W represents an OH or O-G_{S} radical, Y represents a linear or branched alkoxy radical and Z represents H;
it being understood that at least one of W, X, Y or Z represents an O-G_{S} radical.

3. Product according to claim 1 or 2, **characterized in that** X represents an OH or O-G_{S} radical, G_{S}-OH representing a mono- or a disaccharide, or one of their derivatives or analogues, and:
- either W represents an OH or O-G_{S} radical, Y represents H and Z represents H;
- or W represents an OH or O-G_{S} radical, Y represents an OH or O-G_{S} radical and Z represents H;
- or W represents an OH or O-G_{S} radical, Y represents a linear or branched alkoxy radical and Z represents H;
it being understood that at least one of W, X, Y or Z represents an O-G_{S} radical.

4. Product according to claim 1, 2 or 3, **characterized in that** G_{S} is chosen such that G_{S}-OH belongs to the group composed of abequose, rhamnose, arabinose, ribose, xylose, 2-deoxyribose, glucose, galactose, mannose, 2-deoxyglucose, fructose, fucose, N-acetylglucosamine, N-acetylallosamine, galactosamine, mannosamine, saccharose lactose, maltose, cellobiose and trehalose.

5. Product according to any one of claims 1 to 4, **characterized in that** G_{S} is chosen such that G_{S}-OH belongs to the group composed of glucose and lactose.

6. Product according to claim 1, **characterized in that** it is chosen from:
- 3-O-(β-D-glucopyranosyl)ginkgolide A;
- 1-O-(β-D-glucopyranosyl)ginkgolide B;
- 1-O-(α-D-glucopyranosyl)ginkgolide B;
- 10-O-(β-D-glucopyranosyl)ginkgolide B;
- 10-O-(α-D-glucopyranosyl)ginkgolide B;
- 3-O-(β-D-glucopyranosyl)-10-O-(α-D-glucopyranosyl)ginkgolide B;
- 3,10-O-(β-D-glucopyranosyl)ginkgolide B;
- 1,3-O-(β-D-glucopyranosyl)ginkgolide B;
- 1,3,10-O-(β-D-glucopyranosyl)ginkgolideB; and
- 3-O-[4-O-(β-D-galactopyranosyl)-β-D-glucopyranosyl]ginkgolide A.

7. As a medicament, a product according to any one of claims 1 to 6.

8. Pharmaceutical composition comprising, as active ingredient, at least one product according to claim 7.

9. Use of a product according to one of claims 1 to 6 for producing a medicament intended to treat vascular diseases, in particular cardiovascular diseases.

10. Process for the preparation of a product according to one of claims 1 to 6, **characterized in that** it comprises principally a glycosylation stage, which consists of a reaction of a compound of general formula (**III**) represented below, 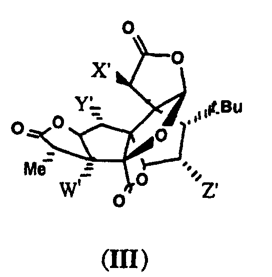 in which W', X', Y' and Z' independently represent an H, OH, linear or branched alkoxy or O-G_{X} radical, G_{X} being a protective group of a hydroxy group preferably being able to be eliminated in a neutral or basic medium, it being understood that at least one of W', X', Y' and Z' represents OH,
with a glycosyl diazirine of general formula (**II**) represented below, 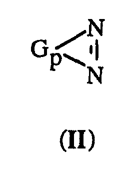 said process also comprising one or more protection and/or deprotection stages of the hydroxy groups, and compound (**II**) being a diazirine derivative of a sugar Gₚ-OH, all the hydroxy groups of which, except that carried by the anomeric carbon, have been protected, for example by benzyl or silyl radicals, the hydroxy group in anomeric position and the hydrogen carried by the same carbon atom having been substituted by an azi group.
